# EUROPÄISCHE PATENTSCHRIFT

(11) **EP 1 678 130 B1**
(45) Veröffentlichungstag und Bekanntmachung des Hinweises auf die Patenterteilung: **20.08.2008**
(21) Anmeldenummer: 04817204.3
(22) Anmeldetag: 14.10.2004
(51) Int. Cl.: C07D 201/00

(54) **AMIDOMETHYL-SUBSTITUIERTE 2-(4-SULFONILAMINO)-3-HIDROXY-3,4-DIHYDRO- 2H-CHROMEN-6-YL-DERIVATE, VERFAHREN UND ZWISCHENPRODUKTE ZU IHRER HERSTELLUNG UND DIESE VERBINDUNGEN ENTHALTENDE ARZNEIMITTEL**
AMIDOMETHYL-SUBSTITUTED 2-(4-SULFONILAMINO)-3-HIDROXY-3,4-DIHYDRO-2H-CHROMIUM-6-YL-DERIVATIVES AND DRUGS CONTAINING SAID COMPOUNDS
DERIVES DE 2-(4-SULFONYLAMINO)-3-HYDROXY-3,4-DIHYDRO-2H-CHROME-6-YL SUBSTITUES PAR UN AMIDOMETHYLE, ET MEDICAMENTS RENFERMANT CES COMPOSES

(30) Priorität: 17.10.2003 DE 10348298
(43) Veröffentlichungstag der Anmeldung: 12.07.2006
(73) Patentinhaber: Solvay Pharmaceuticals GmbH, 30173 Hannover (DE)
(72) Erfinder: SYKES, David, Oxfordshire OX11 7RY (GB); MOLONEY, Brian, Oxfordshire OX11 7US (GB); MARRISON, Lester, Oxfordshire OX11 9RH (GB); ZIEGLER, Dieter, 30966 Hemmingen (DE); MLINARIC, Michael, 30539 Hannover (DE); BOECKER, Christiane, 30519 Hannover (DE); BRUECKNER, Reinhard, 30559 Hannover (DE); WESKE, Michael, 31303 Burgdorf (DE); WITTE, Klaus, 30171 Hannover (DE); FISCHER, Yvan, 30890 Barsinghausen (DE)
(74) Vertreter: Bauriegel, Lutz
(86) Internationale Anmeldenummer: PCT/EP2004/052539
(87) Internationale Veröffentlichungsnummer: WO 2005/037780

(56) Entgegenhaltungen:
- WO-A-00/12077
- US-A- 5 811 448
- US-B1- 6 511 977
- PATENT ABSTRACTS OF JAPAN Bd. 2000, Nr. 15, 6. April 2001 (2001-04-06) & JP 2000 336085 A (NISSAN CHEM IND LTD), 5. Dezember 2000 (2000-12-05)

## Beschreibung

Die vorliegende Erfindung betrifft neue, Amidomethyl-substituierte 2-(4-Sulfonyl-amino)-3-hydroxy-3,4-dihydro-2*H*-chromen-6-ylderivate mit Kaliumkanal-blockierender Wirkung, insbesondere mit das Herz-Kreislaufsystem beeinflussender Wirkung, sowie diese Verbindungen enthaltende Arzneimittel. Ferner betrifft die Erfindung ein Verfahren zur Herstellung der neuen Verbindungen und Zwischenprodukte dieses Verfahrens.

Aus der Schrift WO 00/12077 A1 (entspricht US 6,150,356) sind bereits Indane, Benzopyrane sowie Analoga derartiger Verbindungen bekannt, welche Kaliumkanal-blockierende, insbesondere das Herz-Kreislaufsystem günstig beeinflussende Wirkungen aufweisen.

Die Schrift WO 00/58300 A1 offenbart Chroman-Derivate, welche als Arzneimittel, insbesondere als antiarrhythmisch wirksame Arzneimittel geeignet sind.

Der vorliegenden Erfindung lag die Aufgabe zugrunde, neue Wirkstoffe zur Behandlung von insbesondere Herz-Kreislaufkrankheiten, vorzugsweise von Herzarrhythmien zur Verfügung zu stellen, welche sich durch hohe Wirksamkeit bei guter Verträglichkeit und im Falle antiarrhythmischer Wirkung auch durch ein ausgeprägtes atrialselektives Wirkprofil auszeichnen.

Es wurde nun überraschenderweise gefunden, daß eine erfindungsgemäße Gruppe von neuen Amidomethyl-substituierten 2-(4-Sulfonylamino)-3-hydroxy-3,4-dihydro-2H-chromen-6-ylderivaten Kaliumkanal-blockierende Eigenschaften aufweisen und zur Behandlung von Herz-Kreislaufkrankheiten, vorzugsweise zur Behandlung von Herzarrhythmien geeignet sind. Die erfindungsgemäßen Verbindungen zeichnen sich durch hohe Wirksamkeit bei guter Verträglichkeit und im Falle antiarrhytmischer Wirkung auch durch ein ausgeprägtes atrialselektives Wirkprofil aus. Daneben weisen die erfindungsgemäßen Verbindungen Eigenschaften auf, die eine das Immunsystem beeinflussende Zusatzwirkung erwarten lassen.

Gegenstand der Erfindung sind neue Amidomethyl-substituierte 2-(4-Sulfonyl-amino)-3-hydroxy-3,4-dihydro-2*H*-chromen-6-ylderivate der allgemeinen Formel I, 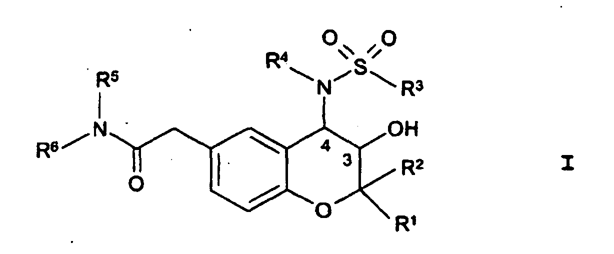 worin,
- R¹: C₁₋₄-Alkyl bedeutet,
- R²: C₁₋₄-Alkyl bedeutet,
- R³: Phenyl, welches gegebenenfalls 1-2-fach durch Halogen, C₁₋₄-Alkyl, C₁₋₄-Alkoxy oder Trifluormethyl substituiert ist; Naphthyl oder Biphenyl bedeutet,
- R⁴: Wasserstoff; C₁₋₆-Alkyl oder C₃₋₇-Cycloalkyl-C₁₋₄-alkyl bedeutet,
- R⁵: Wasserstoff bedeutet und
- R⁶: C₁₋₆-Alkyl; Phenyl-C₁₋₄-alkyl, dessen Phenylgruppe gegebenenfalls 1-fach durch Halogen substituiert ist; Furyl-C₁₋₄-alkyl oder Tetrahydronaphthyl bedeutet, oder
- R⁵ und R⁶: gemeinsam mit dem Stickstoff, woran sie gebunden sind, einen Piperazinring bilden, welcher gegebenenfalls durch Phenyl substituiert ist.

Weiterhin sind Gegenstand der Erfindung die Verbindungen der Formel I enthaltenden Arzneimittel. Ferner sind Gegenstand der Erfindung ein Verfahren zur Herstellung der Verbindungen der Formel I und Zwischenprodukte dieses Verfahrens.

Sofern in den Verbindungen der Formel I oder in anderen im Rahmen der vorliegenden Erfindung beschriebenen Verbindungen Substituenten C₁₋₄-Alkyl oder C₁₋₆-Alkyl bedeuten oder enthalten, kann dieses jeweils geradkettig oder verzweigt sein.

R¹ und R² haben vorzugsweise jeweils die Bedeutung Methyl.

R³ hat vorzugsweise die Bedeutung Phenyl, welches gegebenenfalls 1-2-fach durch Halogen, C₁₋₄-Alkyl, C₁₋₄-Alkoxy oder Trifluormethyl substituiert ist. Insbesondere hat R³ die Bedeutung 1-fach durch C₁₋₄-Alkyl substituiertes Phenyl. Sofern R³ durch Halogen substituiertes Phenyl bedeutet, kommen Fluor, Chlor, Brom und Iod als Halogen in Frage. In einer besonders bevorzugten Bedeutung steht R³ für 4-Ethylphenyl.

R⁴ bedeutet vorzugsweise Wasserstoff, C₁₋₆-Alkyl oder Cyclopropyl-C₁₋₄-alkyl, insbesondere Cyclopropylmethyl. Sofern R⁴ für C₁₋₆-Alkyl steht, ist dieses insbesondere verzweigt und stellt vorzugsweise Neopentyl, 2,2-Dimethylbutyl, 2-Ethylbutyl, 3-Methylbutyl oder 2-Methylpropyl dar.

R⁵ hat vorzugsweise die Bedeutung Wasserstoff.

R⁶ hat vorzugsweise die Bedeutungen Phenyl-C₁₋₄-alkyl, insbesondere Benzyl oder Phenethyl, oder die Bedeutung Tetrahydronaphthyl, insbesondere 1-Tetrahydronaphthyl. (R)-1-Tetrahydronaphthyl ist bevorzugt

Besonders bevorzugte Verbindungen der Formel I sind ausgewählt aus der Gruppe bestehend aus 2-(4-{[(4-Ethylphenyl)sulfonyl]amino}-3-hydroxy-2,2-dimethyl-3,4-dihydro-2*H*-chromen-6-yl)-*N*-1,2,3,4-tetrahydronaphthalen-1-ylacetamid; 2-((3S,4R)-4-{[(4-Ethylphenyl)sulfonyl]amino}-3-hydroxy-2,2-dimethyl-3,4-dihydro-2H-chromen-6-yl)-N-[(1R)-1,2,3,4-tetrahydronaphthalen-1-yl]acetamid; *N*-Benzyl-2-{4-[[(4-ethylphenyl)sulfonyl]-(neopentyl)amino]-3-hydroxy-2,2-dimethyl-3,4-dihydro-2*H*-chromen-6-yl}acetamid; 2-{4-[[(4-Ethylphenyl)sulfonyl](neopentyl)amino]-3-hydroxy-2,2-dimethyl-3,4-dihydro-2*H-*chromen-6-yl}-*N-*(2-phenylethyl)acetamid und 2-(4-{[(4-Methylphenyl)sulfonyl]amino}-3-hydroxy-2,2-dimethyl-3,4-dihydro-2*H*-chromen-6-yl)-*N*-1,2,3,4-tetrahydronaphthalen-1-ylacetamid.

Erfindungsgemäß werden die neuen Verbindungen der Formel I erhalten, indem man eine Verbindung der allgemeinen Formel II, 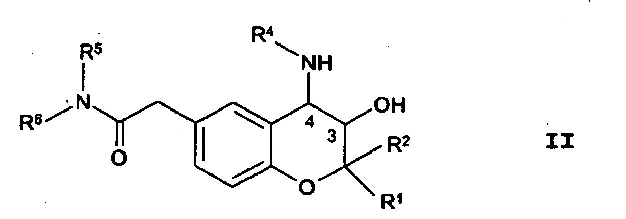 worin R¹, R², R⁴, R⁵, und R⁶ obige Bedeutungen besitzen, mit einer Verbindung der allgemeinen Formel III,

X—SO₂-R³ III

worin R³ obige Bedeutung besitzt und X eine abspaltbare Fluchtgruppe bedeutet, umsetzt. Die Reaktion kann auf üblichem naßchemischem Wege in einem unter den Reaktionsbedingungen inerten organischen Lösungsmittel, insbesondere einem dipolar-aprotischen Lösungsmittel wie Dichlormethan oder in einem Gemisch solcher Lösungsmittel und in Anwesenheit einer Base durchgeführt werden. Als Basen eignen sich nicht-nucleophile organische Stickstoffbasen wie tertiäre Niederalkylamine, beispielsweise Triethylamin. Im Überschuß eingesetzte flüssige organische Basen können auch als Lösungsmittel dienen. Gewünschtenfalls kann die Reaktion durch ein an sich bekanntes Kopplungshilfsmittel wie 4-N,N-Dimethylaminopyridin (= DMAP) katalysiert werden. Geeignete Reaktionstemperaturen liegen zwischen Raumtemperatur und 80°C, beispielsweise bei 65°C. Geeignete Reaktionsdrücke liegen zwischen Normaldruck und etwa 200 bar, beispielsweise bei 180 bar. Sofern die eingesetzte Verbindung der Formel III flüssig ist, kann es vorteilhaft sein, das eingesetzte Lösungsmittel nach der Zugabe der Verbindung der Formel III zu der im Lösungsmittel gelösten Verbindung der Formel II auf an sich bekannte Weise, beispielsweise unter vermindertem Druck, aus dem Reaktionsgemisch zu entfernen. Sofern in den Ausgangsverbindungen der Formel II R⁴ für Wasserstoff steht, ist es zweckmäßig, äquimolare Mengen an Verbindung der Formel **III** einzusetzen. Als Fluchtgruppe X in Verbindungen der Formel **III** wird üblicherweise Halogen, vorzugsweise Chlor oder Brom, eingesetzt. Ferner kann die Umsetzung einer Verbindung der Formen II mit einer Verbindung der Formel III auch in an sich bekannter Weise an fester Phase, insbesondere an einem Reaktionsharz wie Aminomethyl Polystyrol (AMPS) ausgeführt werden. Diese Reaktionsvariante kann vorzugsweise zur Herstellung kleinerer Substanzmengen, beispielsweise im Maßstab von 1 bis 10 mmol, eingesetzt werden. Sofern an fester Phase synthetisiert wird, kann als Base vorzugsweise eine leicht filtrierbare Base wie an sich bekanntes polymergebundenes Methylpiperidin (= Polymer Supported Methylpiperidine, PS-Methylpiperidin) eingesetzt werden. Geeignete Reaktionstemperaturen liegen bei der Festphasensynthese zwischen 10 °C und 40°C, vorzugsweise bei Raumtemperatur. Verbindungen der Formel I können auf an sich bekannte Weise aus dem Reaktionsgemisch isoliert und nötigenfalls auf an sich bekannte Weise gereinigt werden.

Verbindungen der Formel II können hergestellt werden, indem man eine EpoxidVerbindung der allgemeinen Formel IV, 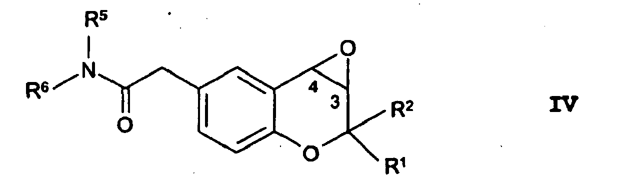 worin R¹, R², R⁵ und R⁶ obige Bedeutungen besitzen, auf an sich bekannte Weise mit einer nucleophilen organischen Stickstoffverbindung, vorzugsweise Ammoniak in wäßriger Lösung, in einem dipolar-protischen Lösungsmittel wie einem Niederalkylalkohol, vorzugsweise Ethanol, umsetzt. Geeignete Reaktionstemperaturen liegen zwischen Raumtemperatur und 60 °C. Sofern Verbindungen der Formel II gewünscht sind, worin R⁴ für C₁₋₆-Alkyl oder C₃₋₇-Cycloalkyl-C₁₋₄-alkyl steht, kann die erhaltene Verbindung der Formel II, worin R⁴ für Wasserstoff steht, anschließend auf an sich bekannte Weise alkyliert werden. Die Alkylierung kann insbesondere als Aminoalkylierung durchgeführt werden, indem man die Verbindung der Formel II, worin R⁴ für Wasserstoff steht, zuerst mit einem Aldehyd der allgemeinen Formel V,

R⁴⁰¹-CHO V

worin R⁴⁰¹ Wasserstoff, C₂₋₅-Alkyl oder C₃₋₇-Cycloalkyl-C₀₋₃-alkyl bedeutet, umsetzt und das entstandene Imin-Zwischenprodukt anschließend durch Zugabe eines Reduktionsmittels zur Alkylaminverbindung der Formel II reduziert. Als Reduktionsmittel eignen sich komplexe Borhydride wie NaBH₃CN oder an sich bekanntes polymergebundenes Borhydrid (= PS-BH₄ ). In einer ersten Variante kann die Reaktion in einem unter den Reaktionsbedingungen inerten polar-protischen organischen Lösungsmittel, insbesondere Methanol, durchgeführt werden, wobei die Reduktion des Imins *in situ* ohne dessen Isolierung in demselben Lösungsmittel durchgeführt wird. Geeignete Reaktionstemperaturen liegen für diese Variante zwischen Raumtemperatur und 60 °C, beispielsweise bei 50 °C. In einer zweiten Variante kann die Umsetzung der Verbindung der Formel II, worin R⁴ für Wasserstoff steht, mit einem Aldehyd der Formel V zu dem Imin-Zwischenprodukt in einem dipolar-aprotischen Lösungsmittel, insbesondere Tetrahydrofuran (= THF), durchgeführt werden. Hierbei ist es vorteilhaft, zur Beschleunigung der Reaktion katalytische Mengen eines wasserziehenden Mittels, beispielsweise eines Orthoesters, insbesondere Trimethylorthoformat (= TMOF), zuzusetzen. Anschließend kann das Imin-Zwischenprodukt isoliert und in einem vorstehend für die erste Variante angegebenen polar-protischen Lösungsmittel aufgenommen werden, um in diesem Lösungsmittel die Reduktion durchzuführen. Diese zweite Variante kann vorzugsweise bei Raumtemperatur durchgeführt werden. Bei der vorstehend in zwei Varianten beschriebenen nucleophilen Ringöffnungsreaktion von Epoxiden der Formel IV werden in aller Regel Verbindungen der Formel II erhalten, worin die vicinalen Substituenten in 3-Stellung und in 4-Stellung des Pyranringes, nämlich die Hydroxygruppe und die Aminogruppe, jeweils in trans-Stellung zueinander stehen.

Verbindungen der Formel II sind neue Verbindungen, welche sich in vorteilhafter Weise als Zwischenprodukte zur Herstellung neuer pharmakologisch aktiver Wirkstoffe, beispielsweise zur Herstellung der Verbindungen der Formel I eigenen.

Verbindungen der Formel III und Verbindungen der Formel V sind an sich bekannt oder können auf an sich bekannte Weise aus bekannten Verbindungen hergestellt werden.

Verbindungen der Formel IV können hergestellt werden, indem man eine Verbindung der allgemeinen Formel VI, 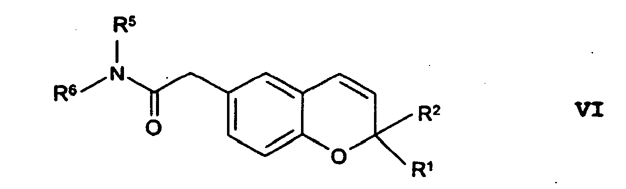 worin R¹, R², R⁵ und R⁶ obige Bedeutungen besitzen, auf an sich bekannte Weise mit einer zur Epoxidbildung befähigten Peroxidverbindung, vorzugsweise mit m-Chlorperbenzoesäure (= MCPBA) in einem unter den Reaktionsbedingungen inerten organischen polar-aprotischen Lösungsmittel, vorzugsweise Dichlormethan, und in Anwesenheit einer Base umsetzt. Als Base eignet sich insbesondere eine wäßrige Lösung von Natriumhydrogencarbonat. Die Reaktion kann vorzugsweise bei Raumtemperatur durchgeführt werden.

Verbindungen der Formel I weisen zumindest in den vicinalen Kohlenstoffatomen in 3-Position und in 4-Position des Pyranringes jeweils ein Chiralitätszentrum auf und können daher in mehreren isomeren Formen auftreten. Gegenstand der Erfindung sind sowohl die isomerenreinen Verbindungen der Formel I, als auch Gemische dieser Isomere. Die optisch aktiven Verbindungen der Formel I können beispielsweise aus den Gemischen der Isomere der Formel I oder aus Gemischen der Isomere der Formel I in an sich bekannter Weise erhalten werden, z. B. durch chromatographische Trennung an chiralen Trennmaterialien. Gemische der Isomere der Formel II können auch durch Umsetzung mit geeigneten optisch aktiven Säuren, z. B. Camphersulfonsäure oder D- bzw. L-Weinsäure, und anschließende Auftrennung in die jeweiligen optischen Antipoden durch fraktionierte Kristallisation der gewonnenen Salze gewonnen werden.

Die optisch aktiven Verbindungen der Formel I können auch durch chirale Synthese direkt hergestellt werden. Sofern Verbindungen der Formel I hergestellt werden sollen, worin der Hydroxysubstituent in 3-Stellung des Pyranringes und der R⁴NSO₂R³-Substituent in 4-Stellung des Pyranringes eine stereochemisch definierte trans-Stellung zueinander aufweisen, kann jeweils von Epoxiden der Formel IV ausgegangen werden, worin die entsprechende Stereochemie bereits vorgebildet ist. Epoxide der Formel IV mit entsprechend vorgebildeter Stereochemie können beispielsweise hergestellt werden, indem man Alkene der Formel VI auf an sich bekannte Weise unter Zuhilfenahme eines chiralen Katalysators nach der Methode von Jacobsen (vgl. z. B. EP 0 521 099 A1) epoxidiert. Sofern beispielsweise eine Verbindung der Formel **I** hergestellt werden soll, worin das Chiralitätszentrum in 3-Position des Pyranringes die S-Konfiguration aufweist und worin das Chiralitätszentrum in 4-Position des Pyranringes die R-Konfiguration aufweist, kann ein Zwischenprodukt der Formel VI in Anwesenheit eines chiralen Katalysators, insbesondere (S,S)-Mangan-III-salen und in Anwesenheit eines Sauerstoffdonors, insbesondere Natriumhypochlorit in wäßriger Lösung, in einem unter den Reaktionsbedingungen inerten organischen Lösungsmittel, insbesondere Dichlormethan, umgesetzt werden. Zweckmäßigerweise wird die Reaktion bei einem pH-Wert zwischen 9,5 und 11,5 durchgeführt. Zur Einstellung eines geeigneten pH-Wertes kann dem Reaktionsgemisch vorzugsweise ein Puffer bestehend aus Na₂HPO₄ und Pyridin-N-oxid zugesetzt werden. Geeignete Reaktionstemperaturen liegen zwischen -10°C und Raumtemperatur, vorzugsweise bei 0 °C. Sofern eine Verbindung der Formel I hergestellt werden soll, worin das Chiralitätszentrum in 3-Position des Pyranringes die R-Konfiguration aufweist und worin das Chiralitätszentrum in 4-Position des Pyranringes die S-Konfiguration aufweist, kann analog zu der vorstehend beschriebenen Vorschrift verfahren werden, wobei man jedoch anstelle des (S,S)-Mangan-III-salens nun das (R,R)-Mangan-III-salen einsetzt.

Verbindungen der Formel VI können hergestellt werden, indem man eine Verbindung der allgemeinen Formel VII, 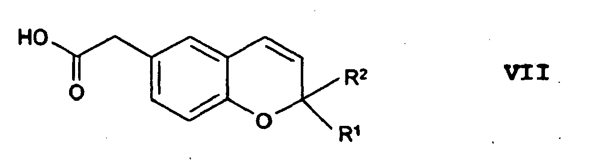 worin R¹ und R² obige Bedeutungen besitzen, mit einer Verbindung der allgemeinen Formel VIII,

HNR⁵R⁶ VIII

worin R⁵ und R⁶ obige Bedeutungen besitzen, auf an sich zur Aminoacylierung bekannte Weise umsetzt. Als Acylierungsmittel können die Carbonsäuren der Formel VII oder deren reaktionsfähige Derivate wie Säurehalogenide, insbesonder Säurechloride oder Säurebromide, eingesetzt werden. Falls als Acylierungsmittel die Säuren der Formel VII selbst eingesetzt werden, kann deren Umsetzung mit den Aminoverbindungen der Formel VIII zweckmäßig auch in Gegenwart eines an sich bekannten Kopplungsreagenzes, beispielsweise 1,1-Carbonyldiimidazol, Chlorameisensäureethylester oder eines Alkylcarbodiimids, z. B. N'-(3-Dimethylaminopropyl)-N-ethylcarbodiimid (= EDC), oder eines Cycloalkylcarbodiimids wie Dicyclohexylcarbodiimid, durchgeführt werden. Die Acylierung kann in einem unter den Reaktionsbedingungen inerten organischen Lösungsmittel bei Temperaturen von -30° bis +50 °C, vorzugsweise bei Raumtemperatur erfolgen. Als Lösungsmittel eignen sich halogenierte Kohlenwasserstoffe wie Dichlormethan oder cyclische Ether wie Tetrahydrofuran oder Dioxan oder Gemische dieser Lösungsmittel.

Verbindungen der Formel VII können hergestellt werden, indem man die Estergruppe einer Verbindung der allgemeinen Formel IX, 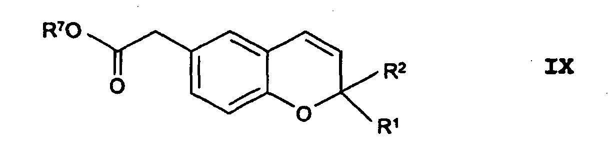 worin R¹ und R² obige Bedeutungen besitzen und R⁷ C₁₋₄-Alkyl bedeutet, auf an sich bekannte Weise verseift. Die Verseifung kann beispielsweise in einem polar-protischen Lösungsmittel wie Ethylenglykol durch kontaktieren mit einer Base, beispielsweise einer starken Base wie verdünnte wäßrige Natronlauge, durchgeführt werden. Geeignete Reaktionstemperaturen liegen zwischen Raumtemperatur und dem Siedepunkt des Lösungsmittels oder des Lösungsmittelgemisches.

Verbindungen der Formel IX können hergestellt werden, indem man eine Verbindung der allgemeinen Formel X, 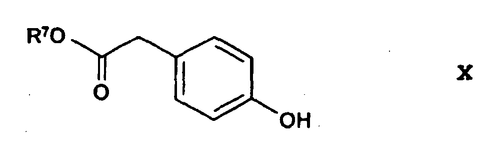 worin R⁷ obige Bedeutung besitzt, mit einer Verbindung der allgemeinen Formel XI, 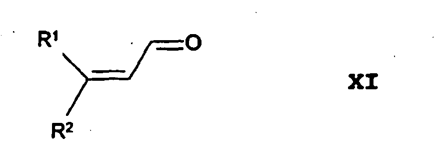 worin R¹ und R² obige Bedeutungen besitzen, auf an sich bekannte Weise umsetzt. Die Umsetzung kann in einem unter den Reaktionsbedingungen inerten organischen Lösungsmittel wie Toluol oder Xylol und in Anwesenheit einer Säure unter Wasserabscheidung durch azeotrope Destillation durchgeführt werden. Als Säure eignet sich beispielsweise Essigsäure oder Propionsäure. Vorteilhaft wird unter Zusatz eines Katalysators wie einer Lewis-Säure, beispielsweise Phenylboronsäure gearbeitet. Geeignete Reaktionstemperaturen liegen zwischen Raumtemperatur und dem Siedepunkt des Lösungsmittels oder Lösungsmittelgemisches, beispielsweise um 120 °C.

Die Verbindungen der Formel X und der Formel XI sind an sich bekannt oder können auf an sich bekannte Weise aus bekannten Verbindungen hergestellt werden.

Die vorteilhaften Wirkungen von Verbindungen der Formel I als pharmakologisch aktive Wirkstoffe werden vor folgendem Hintergrund ersichtlich: Es ist bereits bekannt, daß Substanzen, welche körpereigene kardiale Kaliumkanäle, blockieren, als Wirkstoffe gegen Herz-Kreislaufkrankheiten, insbesondere gegen kardiale Arrhythmien eingesetzt werden können. Durch die Blockade auswärts gerichteter Kaliumströme am Herzen kann eine Verlängerung der Aktionspotentiale des Herzens herbeigeführt werden, welche sich auf arrhythmische Herzzustände günstig auswirkt. Als Beispiele für diesen bekannten Therapieansatz können die Klasse-III-Antiarrhythmika genannt werden. Ein Problem solcher unspezifischen Kaliumkanalblocker ist deren geringe Selektivität bezüglich ihrer Wirkung auf verschiedene Herzgewebe. So wird seit längerer Zeit angenommen, daß insbesondere Klasse-III-Antiarrhythmika zu unerwünschten Verlängerungen des QT-Intervalls im Elektrokardioramm (= EKG) und zu polymorphen ventrikulären Tachykardien ("torsades de pointes") führen können, wodurch letztlich unerwünschte Komplikationen wie beispielsweise Kammerflimmem ausgelöst werden können. Es wurden daher Kaliumkanalblocker gesucht, welche in der Lage sind, selektiv die Kaliumströme des Herzvorhofs (= Atrium), nicht aber der Herzkammer (= Ventrikel) zu beeinflussen. Da die vor einiger Zeit entdeckten Kᵥ1.5-Kaliumkanäle im Herzen ausschließlich im Vorhof, nicht aber im Ventrikel lokalisiert sind, kann von diese Kᵥ1.5-Kaliumkanäle blockierenden Verbindungen angenommen werden, daß sie als atrialselektive Antiarrhythmika geeignet sind. Kᵥ1.5-Kaliumkanäle und andere Kaliumkanäle sind jedoch nicht nur im Herzen, sondern z. B. auch in Körpergefäßen lokalisiert. Daher kann nicht immer ausgeschlossen werden, daß Kᵥ1.5-Kaliumkanal blockierende Verbindungen aufgrund Blockade von Kaliumkanälen in den Gefäßen zu Blutdruckanstiegen führen können. Kᵥ1.5-Kaliumkanal blockierende Verbindungen, welche frei sind von blutdrucksteigernden Begleitwirkungen, sind daher bevorzugt. Weitere unerwünschte Begleitwirkungen, welche bei Verabreichung mancher Kᵥ1.5- Kaliumkanal blockierend wirksamer Verbindungen auftreten können, sind zusätzliche Klasse-I-antiarrhythmische Begleitwirkungen sowie negativ inotrope Effekte.

Die Verbindungen der Formel I zeichnen sich durch eine besonders ausgeprägt und selektiv die kardialen Kᵥ 1.5 - Kaliumkanäle blockierende Wirkung aus. Neben einer besonders guten Wirksamkeit und einem ausgeprägten atrialselektiven antiarrhythmischen Wirkprofil weisen die Verbindungen der Formel I allenfalls geringe unerwünschten Begleitwirkungen wie Blutdruckanstieg, Klasse-I-antiarrhythmische Begleitwirkungen sowie negativ inotrope Effekte auf. Die Verbindungen der Formel I sind daher angezeigt zur Behandlung und/oder Prophylaxe von Herz-Kreislauferkrankungen, insbesondere von Vorhofflimmem, Vorhofflattem und anderen kardialen Arrhythmien, bei größere Säugetieren und Menschen.

Weiterhin zeigen die Verbindungen der Formel I eine deutliche, die Kᵥ1.3-Kaliumkanäle blockierende Wirkung. Kᵥ1.3-Kaliumkanäle sind vorzugsweise in Zellen des Immunsystems lokalisiert. Eine Blockade der Kᵥ1.3-Kaliumkanäle wird unter anderem mit einer antiproliferativen und/oder immunsuppressiven Wirkung in Zusammenhang gebracht (vgl. C. Beeton et al., The Journal of Immunology 166 (2001) 936-944). Von Verbindungen, welche in der Lage sind, Kᵥ1.3-Kaliumkanäle zu blockieren - beispielsweise den Verbindungen der Formel I - kann daher angenommen werden, daß sie sich auch zur Behandlung und/oder Prophylaxe von proliferativen, chronisch entzündlichen und Autoimmunerkrankungen wie der Multiplen Sklerose eignen.

### Beschreibungder pharmakologischen Testmethoden

Die angegebenen Beispielsnummem beziehen sich auf die nachstehend beschriebenen Herstellungsbeispiele.

### 1. In vitro-Untersuchunn der Kᵥ1.5-Kaliumkanal-blockierenden Wirkung der Substanzen

Die Kᵥ1.5-Kaliumkanal-blockierende Wirkung der Substanzen wird in einem an sich bekannten Testmodell oder analog zu diesem Testmodell nachgewiesen (vgl. W. Hu et al., J. Pharmacol. Toxicol. Methods 34 (1995) 1-7). In diesem Testmodell wird eine Zell-linie von ovarzellen des chinesischen Hamsters (= "chinese hamster ovary", "CHO") eingesetzt, welche aus einer einzigen Zelle stammt und stabil den Kᵥ1.5-Kanal exprimiert. Durch Inkubation über Nacht in einem RbCl enthaltenden Nährmedium oder einem "Beladungspuffer" (alle Werte in mM: RbCl 5, NaCl 140, CaCl₂ 2, MgSO₄ 1, HEPES-Puffer 10, Glucose 5) werden die vorgenannten ovarzellen unter dem Einfluß der Na⁺/K⁺ ATPase mit Rb⁺ beladen. Danach wird ein Teil der Eizellen als Vergleichsstandard in Abwesenheit eines Inhibitors inkubiert, während ein anderer Teil der ovarzellen in Anwesenheit der jeweiligen inhibitorisch wirksamen Testsubstanz der Formel I inkubiert wird. Anschließend werden die ovarzellen durch Erhöhung der extrazellulären Kaliumionenkonzentration depolarisieht, wodurch die Kᵥ1.5-Kaliumkanäle der ovarzellen sich öffnen. In Abwesenheit eines Inhibitors fließen die Rb⁺-Ionen durch die Kᵥ1.5-Kaliumkanäle in die sie umgebende Flüssigkeit. In Anwesenheit einer inhibitorisch wirksamen Testsubstanz der Formel I bleiben die Rb⁺-Ionen dagegen im Inneren der ovarzellen eingeschlossen. Das Ausmaß der Kᵥ1.5-Kaliumkanal-blockierenden Wirkung der Testsubstanzen der Formel I wird durch Messung der Rb⁺-Ionenkonzentration in der sie umgebenden Flüssigkeit mittels Atomabsorptionsspektroskopie gegen einen Vergleichsstandard bestimmt.

Ovarzellen des chinesischen Hamsters (siehe oben) wurden in einem an sich bekannten, RbCl enthaltenden Nährmedium für CHO-Zellen kultiviert und auf die Probenplätze einer 96 Proben fassenden Probenplatte ("96 well plate") gegeben. Man ließ die Ovarzellen über Nacht wachsen, um Monolagen der Ovarzellen erhalten. Dann wurde zu-nächst das Nährmedium abpipettiert und jeder Probenplatz wurde mit je dreimal 100 µl eines Präinkubationspuffers niedriger Kaliumionenkonzentration (alle Werte in mM: KCl 5, NaCl 140, CaCl₂ 2, MgSO₄ 1, HEPES-Puffer 10, Glucose 5) gewaschen. Anschließend gab man zu jedem Probenplatz je 50 µl einer Lösung der jeweiligen Testsubstanz Stammlösung in DMSO, Verdünnung mit Präinkubationspuffer, Endkonzentration im Versuchsansatz 10 µM) oder des Lösungsmittels (als Negativkontrollen) zu und inkubierte je 10 Min. lang bei Raumtemperatur. Anschließend wurden zu jedem Probenplatz je 50 µl eines Stimulationspuffers mit erhöhter Kaliumionenkonzentration (KCI 145 mM, NaCl 0 mM, sonst wie Präinkubationspuffer) zugegeben und die Proben dann weitere 10 Min. lang bei Raumtemperatur inkubiert. Je 80 µl der die ovarzellen umgebenden Flüssigkeit aus jedem Probenplatz wurden nun jeweils getrennt voneinander auf die Probenplätze einer Analyse-Probenplatte überfährt, und es wurde in den Flüssigkeiten durch Atomabsorptionsspektroskopie die Rb⁺-Ionenkonzentration bestimmt Die Testsubstanzen wurden jeweils doppelt getestet. Der Signal-Ausschnitt, welcher die Kᵥ1.5-Komponente des Rb⁺-Ausstromes repräsentierte, wurde definiert, indem man als Positivkontrolle den an sich bekannten Kaliumkanalblocker 4-AP in einer hohen Konzentration (100 X IC₅₀ für den Kᵥ1.5Kanal) verwendete. Dadurch konnte ermittelt werden, welcher Anteil des Rb⁺-Ausstroms vom Einfluß des 4-AP abhängig war und daher dem Kᵥ1.5-Kanal zuzuordnen ist. Bei den Substanzen, die in der verwendeten Konzentration von 10 µM zu einer Abnahme des Rb⁺-Ausstroms um mindestens 50 % führten, wurden zusätzliche Versuche mit niedrigeren Konzentrationen der Testsubstanz durchgeführt, um die halbmaximal wirksame Konzentration bestimmen zu können. Als Kenngröße wurde jeweils die Konzentration halbmaximaler Hemmung der Testsubstanz der Formel I (IC₅₀) angegeben.

In diesem Testmodell zeigten die in der nachfolgend aufgeführten Tabelle 1 aufgeführten Testsubstanzen der Formel I die nachfolgend angegebenen IC₅₀-Werte:

**Tabelle 1: Kᵥ1.5-Kaliumkanal-blockierende Wirkung der Testsubstanzen in vitro**

| **Beispiel** Nr. | **IC₅₀** |
|---|---|
| 2 | 2,0 |
| 4 | 1,6 |
| 5 | 5,0 |
| 7 | 1,5 |
| 8 | 0,5 |
| 9 | 2,9 |
| 10 | 1,6 |
| 11 | 3,2 |
| 12 | 3,2 |
| 13 | 6,5 |
| 15 | 4,0 |
| 16 | 6,3 |
| 17 | 2,65 |
| 18 | 2,7 |
| 19 | 2,5 |
| 6 | 2,9 |
| 21 | 33 |
| 22 | 3,4 |
| 23 | 4,3 |
| 24 | 5 |
| 25 | 5,2 |
| 26 | 5,2 |
| 27 | 5,6 |

### 2. In vitro-Untersuchung der Kᵥ1.3-Kaliumkanal-blockierenden Wirkung der Substanzen

Die Kᵥ1.3-Kaliumkanal-blockierende Wirkung der Substanzen wird in einem an sich bekannten Testmodell (z. B. der Firma Genion, Hamburg) oder analog zu diesem Testmodell nachgewiesen (vgl. J. Pläsek und K. Sigler, J. Photochem. Photobiol. 33 (1996) 101-124). In diesem Testmodell werden an sich bekannte ovarzellen des chinesischen Hamsters (= CHO) eingesetzt, welche stabil mit dem Kᵥ1.3-Kaliumkanal transfiziert sind. Die Blockade der zelleigenen Kᵥ1.3-Kaliumkanalaktivität in den transfizierten Zellen geht mit einer positiven Verschiebung des Membranpotentials von ca. -40 mV auf -30 mV einher, während in den parallel untersuchten Wildtyp-CHO-Zellen keine nennenswerte Verschiebung des Membranpotentials ausgelöst wird. Eine Änderung des Membranpotentials hängt somit mit der Verminderung der Kᵥ1.3-Kaliumkanal-Aktivität zusammen. Durch Blockade der Kᵥ1.3-Kaliumkanäle z.B. mit Substanzen der Formel I und daraus resultierender Änderung des Membranpotentials kommt es zu einer Akkumulierung eines Membranpotential-empfindlichen Fluoreszenzfarbstoffes in intrazellulären Kompartimenten der Ovarzellen und letztlich zu einer zunehmenden Fluoreszenz. Die Änderung des Membranpotentials der Ovarzellen wird also indirekt über die Fluoreszenzzunahme der Membranpotential-empfindlichen Farbstoffe gemessen

Die Transfektion der Zellen mit dem Kᵥ1.3-Plasmid erfolgte auf an sich bekannte Weise mit einem kommerziell erhältlichen Transfektionsreagenz (DMRIE-C der Firma Gibco BRL, Deutschland). Die erfolgreiche Transfektion wurde mittels Immunfluoreszenz sowie durch "patch-clamp"-Untersuchungen des Kaliumionenstroms verifiziert. Die Fluoreszenzmessungen wurden auf einem Tecan Safire-Fluoreszenzleser der Fa. Tecan, Deutschland, durchgeführt Als Kenngröße wurde jeweils die Zunahme der Fluoreszenzintensität ermittelt, welche durch Blockade der Kᵥ1.3-Kaliumkanäle in den Ovarzellen mit Substanzen der Formel I in einer Konzentration von 10 µM verursacht wurde. Die Zunahme der Fluoreszenzintensität wurde jeweils angegeben in Prozent (%) gegenüber einer durch die Referenzsubstanz Margatoxin verursachten Zunahme der Fluoreszenzintensität. Margatoxin ist als selektiver Kᵥ1.3-Kaliumkanal-Blocker bekannt (siehe z. B. M. Garcia-Calvo et al., J. Biol. Chem. 268 (1993) 18866-18874).

In diesem Testmodell zeigten die in der nachfolgend aufgeführten Tabelle 2 aufgeführten Testsubstanzen der Formel I die nachfolgend angegebenen %-Werte:

**Tabelle 2: Kᵥ1.3-Kaliumkanal-blockierende Wirkung der Testsubstanzen in vitro**

| **Beispiel Nr.** | **Zunahme der Fluoreszenzintensität (% Margatoxin)** |
|---|---|
| 2 | 72 |
| 6 | 97 |
| 5 | 82 |
| 9 | 107 |
| 10 | 82 |
| 11 | 99 |
| 12 | 111 |
| 13 | 53 |
| 17 | 72 |
| 18 | 141 |
| 19 | 171 |
| | |
| 21 | 86 |
| 22 | 71 |
| 23 | 41 |

### 3. Untersuchung der funktionellen Wirksamkeit der Substanzen am Vorhof des Rattenherzens in vitro

Die funktionelle antiarrhythmische Wirksamkeit der Substanzen wird in dem nachfolgend angegebenen Testmodell nachgewiesen. In diesem Testmodell wird bestimmt, in welchem Ausmaß die Kᵥ1.5-blockierend wirkenden Substanzen der Formel I zu einer Verlängerung der funktionellen Refraktärzeit im linken Herzvorhof der Ratte führen. Die Refraktärzeit ist die kleinstmögliche Zeitspanne zwischen Grundreiz und Zusatzstimulus, in der eine erneute Kontraktion ausgelöst werden kann. Das Ausmaß der Verlängerung der funktionellen Refraktärzeit ist ein Maß für die antiarrhythmische Wirksamkeit der erfindungsgemäßen Substanzen. Die funktionelle Refraktärzeit wird ermittelt, indem man am elektrisch gereizten Präparat prüft, in welchem zeitlichen Abstand zur vorausgegangenen Kontraktion durch elektrische Zusatzreize eine erneute Kontraktion ausgelöst werden kann.

Frisch getöteten Ratten (Sprague-Dawley, Charles-River, Deutschland) wurden die Herzen entnommen. Die linken Herzvorhöfe wurden isoliert und an Kraftaufnehmem in einem temperierten (30 °C), begasten (O₂ 95 %, CO₂ 5 %) Organbad befestigt; welches mit modifizierter Tyrode-Lösung (alle Werte in mM: NaCl 137; KCl 2,7; CaCl₂ 1,8; MgCl₂ 0,8; NaHCO₃ 11,9; NaH₂PO₄ 0,6; Glucose 5) gefüllt war. Um regelmäßige Kontraktionen auszulösen, wurden die Präparate elektrisch gereizt (Rechteckimpulse, Impulsstärke 3,5 x Schwellenreiz, Impulsbreite 1,5 ms, Frequenz 1 Hz). Zunächst wurde der Ausgangswert der funktionellen Refraktärzeit bestimmt, indem zusätzlich zum Grundreiz Extraimpulse appliziert wurden, wobei der zeitliche Abstand zum vorangegangenen Grundreiz so lange verkürzt wurden, bis keine Zusatzkontraktion mehr auslösbar waren. Anschließend erfolgte die kumulative Zugabe steigender Konzentrationen (0,1-10 µM) der Substanzen der Formel I in Intervallen von je 20 Min., wobei jeweils 18 Min. nach erfolgter Zugabe erneut die Refraktärzeit bestimmt wurde. Vor der Messung wurden von den Testsubstanzen Stammlösungen (3,2 und 0,32 mM in 100 % DMSO) hergestellt. Um die gewünschten Endkonzentrationen der Substanzen (0,1 - 10 µM) im Organbad (Volumen 100 ml) zu erreichen, wurden dann entsprechende Volumina dieser Stammlösungen in das Organbad gegeben.

Als Kenngröße wurde jeweils die Verlängerung der funktionellen Refraktärzeit (Functional Refractory Period, FRP) im linken Vorhof des Rattenherzens in Millisekunden angegeben, welche nach Zugabe von 10 µM der jeweiligen Substanz der Formel I zu den Herzvorhofpräparaten beobachtet wurde.

In diesem Testmodell zeigten die in der nachfolgend angegebenen Tabelle 3 aufgeführten Testsubstanzen der Formel I die nachfolgend angegebenen Refraktärzeitiverlängerungen, wobei höhere Werte für eine stärkere antiarrhythmische Wirksamkeit stehen:

**Tabelle 3: FRP verlängernde Wirkung der Testsubstanzen (10 µM) am linken Vorhof des Rattenherzens in vitro**

| **Beispiel Nr.** | **FRP-Verlängerung [ms]** |
|---|---|
| 1 | 15 |
| 2 | 20 |
| 4 | 17 |
| 7 | 24 |
| 8 | 17 |
| 9 | 30 |
| 10 | 20 |
| 11 | 24 |
| 12 | 14 |
| 13 | 28 |
| 14 | 22 |
| 15 | 30 |
| 16 | 20 |

### 4. Untersuchung der funktionellen Wirksamkeit der Substanzen am Herzen des Meerschweinchens in vivo

In dem nachfolgend angegebenen Testmodell wird gezeigt, daß die erfindungsgemäßen Substanzen allenfalls geringe unerwünschte proarrhythmische Wirkungen auf die Erregungsrückbildung in der Herzkammer aufweisen. Hierzu wird der Einfluß der Verbindungen der Formel I auf die effektive Refraktärzeit (Effective Refractory Period, ERP) und andere Einflußgrößen des Meerschweinchenherzens in vivo untersucht. In diesem Testmodell führen nicht-erfindungsgemäße nicht-selektive Kaliumkanal-Blocker, welche auch HERG- und/oder KᵥLQT1-Kanäle blockieren, zu einer unerwünschten Verlängerung der ERP sowie der QT-Zeit im Elektokardiogramm (= EKG). Die QT-Zeit ist ebenfalls ein Maß für die Erregungsrückbildung im Herzen. Substanzbedingte Verlängerungen der ERP und der QT-Zeit werden beide jeweils eigenständig als Hinweise auf das Risiko des Auftretens von unerwünschten Torsade-de-Pointes-Arrhythmien gedeutet. Ferner wurde aus dem EKG auch jeweils das QRS-Intervall als Maß der ventrikulären Erregungsausbreitungsgeschwindigkeit ermittelt. Auch eine durch eine Testsubstanz verursachte Verlängerung des QRS-Intervalls wird mit einem erhöhten Risiko unerwünschter proarrhythmischer Begleitwirkungen in Verbindung gebracht. Daher bedeutet in diesem Testmodell das Fehlen einer ERP- und QT-Zeit-Verlängerung ein geringes Risiko, das Auftreten einer relevanten ERP- und QT -Verlängerung dagegen ein erhöhtes Risiko unerwünschter proarrhythmischer Wirkungen. Auch das Fehlen einer substanzbedingten Verlängerung des QRS-Intervalls durch die untersuchten Substanzen der Formel I bezeichnet ein geringes Risiko unerwünschter proarrhythmischer Begleitwirkungen, da eine fehlende QRS-Verlängerung auf eine ungestörte Erregungsausbreitung in der Herzkammer hinweist. Umgekehrt spricht eine QRS-Verlängerung, die typischerweise durch Klasse-I-Antiarrhythmika ausgelöst wird, für eine Verlangsamung der Leitungsgeschwindigkeit und kann das Auftreten von Kammertachykardien bis zum Herzkammerflimmern begünstigen.

Männliche Meerschweinchen (Dunkin-Hartley der Firma Charles River) wurden narkotisiert (Ketamin 50 mg/kg, Xylazin 10 mg/kg) und es wurde ihnen jeweils über die eine Vena jugularis ein venöser Zugang für die Applikation von Verbindungen der Formel I oder eines Vehikels gelegt. Über die andere Vena jugularis wurde den Meerschweinchen ein bipolarer Stimulationskatheter in den rechten Ventrikel vorgeschoben (Stimulationsfrequenz 5 Hz). Der arterielle Blutdruck wurde über einen in der Arteria carotis befindlichen Katheter gemessen, welcher an einen Statham-Drucksensor angeschlossen war. Das EKG wurde über Nadelelektroden abgeleitet. Die Meßdaten wurden über einen A/D-Wandler digitalisiert, auf einem Computer mit geeigneter Software (Ponemah Physiology Platform der Fa. Gould, USA) erfaßt und parallel auf einem Mehrkanalschreiber ausgedruckt. Nach einer Äquilibrierungsperiode von 45 Min. wurden den Meerschweinchen in 12-Min.-Intervallen steigende Dosierungen der Verbindungen der Formel I oder des Vehikels intravenös (= i.v.) appliziert. Vor erster Applikation und jeweils eine Min. nach Applikation steigender Dosierungen (0.1 - max. 30 µmol/kg) der Substanzen der Formel I wurde die effektive Refraktärzeit gemessen. Hierzu wurde nach jeweils fünf normalen Stimulationsreizen ein zusätzlicher Puls appliziert und dessen zeitlicher Abstand vom vorangegangenen Puls so lange gesteigert, bis es zur Auslösung einer Herzaktion kam. Das beobachtete Zeitintervall entspricht der ERP des ventrikulären Myokards.

Um mögliche Effekte der Testsubstanzen auf den Blutdruck zu erfassen, wurde in demselben Testmodell nach jeder Substanzgabe der systolische und diastolische Blutdruck ermittelt und mit dem vorherigen Blutdruckniveau verglichen. Die Parameter wurden automatisch 1 und 8 Min. nach jeder Substanzverabreichung aufgezeichnet. In Tabelle 4 sind weiterhin die Veränderungen des systolischen Blutdrucks durch die nachfolgend angegebenen Verbindungen der Formel I (abzüglich Vehikel-bedingter Effekte) dargestellt. Keine der angegebenen Verbindungen führte zu einer relevanten Blutdruckerhöhung.

In diesem Testmodell zeigten die in der nachfolgend angegebenen Tabelle 4 aufgeführten Testsubstanzen der Formel I die nachfolgend angegebenen Wirkungen. Es wurden nur statistisch signfikante Effekte aufgeführt, wobei ein t-Test mit einer Signifikanzgrenze von *P*<0,05 für die statistische Prüfung verwendet wurde. In der nachfolgend angegebenen Tabelle 4 bedeutet die Angabe "n.s." (= "nicht statistisch signifikant"), daß die Substanz des entsprechenden Beispieles auf die angegebene Meßgröße keinen statistisch signifikanten Einfluß ausübt.

**Tabelle 4: Wirkung der Testsubstanzen (1 Min. nach Applikation von 10 µmol/kg i.v.) auf die ERP, QT- und QRS-Intervalle in der Herzkammer des Meerschweinchens und gleichzeitig gemessene Veränderungen des systolischen Blutdrucks in vivo (n.s. = nicht statistisch signifikant, negative Werte bedeuten Verkürzung bzw. Abnahme)**

| **Bsp.-Nr.** | **ERP (ms)** | **QT (ms)** | **QRS (ms)** | **syst. Blutdruck (mmHg)** |
|---|---|---|---|---|
| 1 | n.s. | n.s. | n.s. | n.s. |
| 2 | n.s. | n.s. | n.s. | n.s. |
| 4 | n.s. | n.s. | n.s. | n.s. |
| 7 | -8,2 | n.s. | n.s. | -15,3 |
| 8 | n.s. | n.s. | n.s. | -10,7 |
| 10 | -8,0 | n.s. | n.s. | -15,9 |
| 11 | n.s. | n.s. | n.s. | n.s. |
| 12 | n.s. | n.s. | n.s. | n.s. |
| 13 | n.s. | n.s. | n.s. | n.s. |
| 16 | -7,5 | n.s. | n.s. | -8.6 |

### 5. Untersuchung der funktionellen Wirksamkeit der Substanzen am Herzen der narkotisierten Katze in vivo

In dem nachfolgend angegebenen Testmodell wird gezeigt, daß die erfindungsgemäßen Substanzen eine ausgeprägte atrialselektive Wirkung am Herzen aufweisen. Nach Verabreichung der erfindungsgemäßen Substanzen wird eine starke Erhöhung der atrialen Flimmerschwelle - also der Stromstärke, bei der es zum Auftreten von Vorhofflimmern kommt- beobachtet. Gleichzeitig wird dagegen die ventrikuläre Flimmer schwelle nur minimal beeinflußt.

Lebende Katzen wurden mit Chloralose-Urethan (50/300 mg/kg i.v.) narkotisiert und mit Raumluft beatmet. Dann wurden nach Thorakotomie Reizelektroden am rechten Vorhof und am Ventrikel angenäht. Die Bestimmung der atrialen und ventrikulären Flimmerschwelle erfolgte auf an sich bekannte Weise durch Applikation von Rechteckimpulsen steigender Stromstärke bis zum Auftreten von Vorhof- bzw. Kammerflimmern (zur Durchführung siehe im Einzelnen: Br. J. Pharmac. 17 (1961) 167; Hdb. exp. Pharmacol. XVI/3 (1975) 131; Pharmacol. Res. 25 Suppl. 2 (1992) 156). Die Testsubstanzen der Formel I wurden in Propylenglykol (80%) gelöst und in steigenden Dosierungen (5 - 30 µmol/kg) intravenös appliziert. Atriale und ventrikuläre Flimmerschwellen wurden dann in 5 min-Intervallen nach Zugabe der jeweiligen Dosierung auf an sich bekannte Weise bestimmt. Die Zunahme der jeweiligen Flimmerschwelle durch die untersuchten Substanzen wurde in Prozent des Wertes vor Substanzgabe ausgedrückt, d. h. eine Verdopplung der Flimmerschwelle entspricht einem Anstieg um 100 %.

In diesem Testmodell zeigten die in der nachfolgend angegebenen Tabelle 5 aufgeführten Testsubstanzen der Formel I die nachfolgend angegebenen Wirkungen.

**Tabelle 5: Erhöhung der atrialen (AFT) und ventrikulären Flimmerschwelle (VFT) in narkotisierten Katzen in vivo (Dosis 30 µmol/kg i.v.)**

| **Bsp.-Nr.** | **AFT (%)** | **VFT (%)** | **Selektivitätsfaktor (AFT:VFT)** |
|---|---|---|---|
| 1 | 66 | 22 | 3 |
| 2 | 261 | 15 | 17 |
| 4 | 226 | 19 | 12 |
| 9 | 213 | 29 | 7 |
| 10 | 241 | 19 | 13 |

Die besonders gute Verträglichkeit der erfindungsgemäßen Verbindungen kann auch in weiteren pharmakologischen Testmodellen gezeigt werden. So kann beispielsweise in einem in vitro-Test an Herzmuskelpräparaten von Meerschweinchen gezeigt werden, daß die Verbindungen der Formel I allenfalls geringe Klasse-I-antiarrhythmische Begleitwirkungen aufweisen. Ferner kann in einem in vitro Modell am Rattenherzen und in einem anderen in vitro-Modell am Meerschweinchenherzen gezeigt werden, daß die Verbindungen der Formel I allenfalls geringe negativ-inotrope Effekte verursachen.

Die Verbindungen der Formel I können in üblichen pharmazeutischen Zubereitungen verabreicht werden. Im Einzelfall können spezielle Dosierungsformen angezeigt sein. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größere Säugetieren jedoch Arzneiformen mit einem Wirkstoffgehalt von 0,2 bis 500 mg, insbesondere 10 bis 200 mg Wirkstoff pro Einzeldosis. Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt, oder auch Suppositorien. Diese Präparate können pharmazeutisch übliche anorganische und/oder organische Trägerstoffe, wie z. B. Talkum, Milchzucker oder Stärke, neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nachfolgend angeführten Beispiele sollen die Erfindung näher erläutern, ohne sie in ihrem Umfang zu beschränken.

### Beispiel 1:

### 2-(4-{[(4-Ethylphenyl)sulfonyl]amino}-3-hydroxy-2,2-dimethyl-3,4-dihydro-2H-chromen-6-yl)-N-1,2,3,4-tetrahydronaphthalen-1-ylacetamid

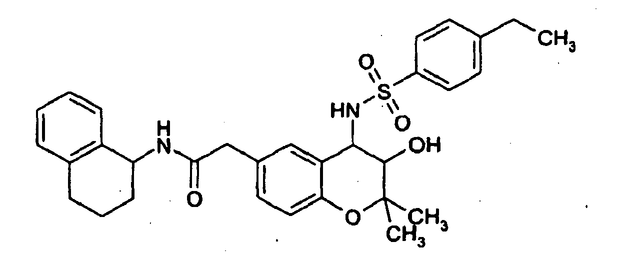

A) 25 g Methyl-4-hydroxyphenylacetat, 14,5 ml 3-Methylbut-2-enal und 18,3 g Phenylboronsäure wurden unter Stickstoffatmosphäre in 1 I trockenem Toluol zusammengegeben und 7 h lang unter Rückflußkühlung zum Sieden erhitzt. Anschließend gab man zu dieser Vorlage bei Raumtemperatur (= RT) 60 ml Eisessig hinzu und ließ erneut 7 h lang unter Rückflußkühlung zum Sieden erhitzen. Man ließ auf RT abkühlen, dampfte unter vermindertem Druck das Lösungsmittel weitgehend ein und goß den verbliebenen Rückstand in 300 ml eines 1:1 (v/v) Gemisches aus Essigsäureethylester (= EE) und Wasser. Man stellte den pH-Wert durch Zugabe von festem Natriumbicarbonat auf 5 ein, trennte die organische Phase ab und dampfte diese unter vermindertem Druck weitgehend ein. Chromatographie des verbliebenen Rückstandes an Kieselgel (mobile Phase: Petrolether/EE 10:1 v/v) lieferte 16 g Methyl-(2,2-dimethyl-2*H*-chromen-6-yl)acetat als blassgelbes Öl, ¹H-NMR (400 MHz, CDCl₃) δ [ppm] : 1.15 (s, 3 H) 1.27 (t, 3 H) 1.43 (s, 3 H) 1.60 - 1.85 (3 H) 1.97 (m, 1 H) 2.66 - 2.82 (4 H) 3.20 - 3.29 (3 H) 3.54 (dd, 1 H) 4.23 (dd, 1 H) 5.06 (m, 1 H) 5.28 (d, 1 H) 5.59 (d, 1 H) 6.55 (d, 1 H) 6.66 (d, 1 H) 6.97 (dd, 1 H) 7.03 - 7.18 (4 H) 7.35 (m, 2 H) 7.87 (m, 2 H); ¹³C-NMR (101 MHz, CDCl₃) δ [ppm] : 15.1 (q) 18.5 (q) 20.1 (t) 26.6 (q) 28.8 (t) 29.2 (t) 30.2 (t) 42.9 (t) 47.7 (d) 55.1 (d) 74.8 (d) 78.7 (s) 117.9 (d) 121.3 (s) 126.3 (d) 127.2 (s) 127.4 (d, 3 C) 128.2 (d) 128.8 (d) 128.9 (d, 2 C) 129.3 (d) 130.3 (d) 136.5 (s) 137.6 (s) 137.7 (s) 150.2 (s) 152.3(s) 170.3 (s).
B) 46 g des vorstehend erhaltenen Methyl-(2,2-dimethyl-2H-chromen-6-yl)acetats (Gesamtmenge aus mehreren analogen Ansätzen), 150 ml Ethylenglykol und 400 ml einer 20 %-igen wäßrigen Natronlauge wurden 3 h lang in 440 ml Tetrahydrofuran (= THF) unter Rückflußkühlung zum Sieden erhitzt. Anschließend ließ man das entstandene Gemisch auf RT abkühlen, dampfte das Lösungsmittel weitgehend unter vermindertem Druck ein, und fügte zu dem verbliebenen Rückstand 200 ml tert.-Butylether und 300 ml Wasser hinzu. Man ließ 10 Min. lang rühren und säuerte dann die wäßrige Phase durch Zugabe einer 20 %-igen wäßrigen Salzsäurelösung auf pH 6 an. Die wäßrige Phase wurde zweimal mit je 300 ml Dichlormethan extrahiert und die vereinigten organischen Phasen wurden über 20 g Natriumsulfat getrocknet. Man dampfte das Lösungsmittel unter vermindertem Druck weitgehend ein, versetzte den verbliebenen Rückstand mit Petrolether und filtrierte eine erste entstandene Kristallfraktion vom Lösungsmittel ab. Das Filtrat wurde erneut unter vermindertem Druck konzentriert, wobei erneut eine Kristallfraktion ausfiel. Die vereinigten Kristallfraktionen wurden getrocknet und man erhielt 33,8 g 2,2-(Dimethyl-2*H* chromen-6-yl)essigsäure, welche ohne weitere Reinigung für die nachfolgende Umsetzung eingesetzt wurde, ¹H-NMR (400 MHz, CDCl₃) δ [ppm] : 1.41 (s, 6 H) 3.52 (s, 2 H) 5.59 (d, 1 H) 6.27 (d, 1 H) 6.72 (d, 1 H) 6.88 (d, 1 H) 6.99 (dd, 1 H); ¹³C-NMR (101 MHz, CDCl₃) δ [ppm] : 28.1 (q, 2 C) 40.2 (t) 76.3 (s) 116.5 (d) 121.4 (s) 122.1 (d) 125.3 (s) 127.2 (d) 129.9 (d) 131.1 (d) 152.3 (s) 177.8 (s).
C) 9,6 g in 85 ml THF gelöstes 1,1-Carbonyldiimidazol (= CDI) wurde langsam zu einer Lösung von 11,7 g der vorstehend erhaltenen 2,2-(Dimethyl-2*H*-chromen-6-yl)essigsäure in 100 ml THF hinzugegeben und 30 Min. lang bei RT gerührt. Zu dieser Vorlage tropfte man langsam 8,8 ml 1,2,3,4-Tetrahydro-1-naphthylamin, gelöst in 30 ml THF, hinzu, rührte das entstandene Gemisch 1 h lang und ließ über Nacht bei RT stehen. Das Lösungsmittel wurde unter vermindertem Druck weitgehend einge-dampft und der verbliebene Rückstand wurde mit einer Mischung aus Diethylether und Isopropanol (100:1 v/v) gerührt und kristallisiert. Die enstandenen Kristalle wurden abgesaugt und bei 65 °C und 20 bar getrocknet. Chromatographie der Diethylether / Isopropanol-Waschflüssigkeit an Kieselgel lieferte weiteres Zwischenprodukt, welches mit der Hauptmenge vereinigt und getrocknet wurde. Man erhielt 17,5 g 2-(2,2-Dimethyl-2*H*-chromen-6-yl)-*N*-1,2,3,4-tetrahydronaphthaten-1-ylacetamid als farblosen Feststoff, welcher ohne weitere Reinigung für die nachfolgende Umsetzung eingesetzt wurde. ¹H-NMR (400 MHz, CDCl₃) δ [ppm] : 1.41 (s, 6 H) 1.601.85 (3H) 1.98 - 2.08 (1 H) 2.65 - 2.80 (2H) 3.45 - 3.55 (2H) 5.12 - 5.20 (1 H) 5.60 (d, 1 H) 5.66 (d, 1 H) 6.26 (d, 1 H) 6.71 (d, 1 H) 6.86 (d, 1 H) 6.96 (dd, 1 H) 7.02 - 7.07 (1 H) 7.08-7.17 (3 H); ¹³C-NMR (101 MHz, CDCl₃) δ [ppm] : 20.2 (t) 28.0 (q, 2 C) 29.2 (t) 30.3 (t) 43.2 (t) 47.7 (d) 76.3 (s) 116.8 (d) 121.7 (s) 122.0 (d) 126.2 (d) 126.9 (s) 127.2 (d) 128.2 (d) 129.1 (d) 129.8 (d) 131.3 (d) 136.7 (s) 137.5 (s) 152.2 (s) 170.7 (s).
D) 950 ml einer gesättigten wäßrigen Natriumhydrogencarbonat-Lösung wurden zu einer Lösung von 11g des vorstehend erhaltenen 2-(2,2-Dimethyl-2H-chromen-6-yl)-N-1,2,3,4-tetrahydronaphthalen-1-ylacetamids in 600 ml Dichlormethan zugegeben. Zu dieser Vorlage gab man insgesamt 15 g m-Chlorperoxybenzoesäure (= MCPBA) in drei Portionen von je 5 g im Abstand von jeweils 5 Min. hinzu und rührte 18 h lang bei RT. Die organische Phase wurde abgetrennt und am Rotationsverdampfer bei 65°C und 20 bar weitestgehend eingedampft. Man erhielt 2-(2,2-Dimethyl-1a,7b-dihydro-2*H*-oxireno[c]chromen-6-yl)-*N*-1,2,3,4-tetrahydronaphthalen-1-ylacetamid als rohes Öl, welches ohne weitere Reinigung für die nachfolgende Umsetzung eingesetzt wurde, ¹H-NMR (400 MHz, CDCl₃) δ [ppm] : 1.23 (s, 6 H) 1.56 (s, 6 H) 1.63-1.85 (6 H) 1.97-2.10 (2 H) 2.65 - 2.82 (4 H) 3.43 - 3.56 (6 H) 3.84 - 3.89 (2 H) 5.12 - 5.22 (2 H) 5.62 -5-72 (2 H) 6.75 (d, 1 H) 6.76 (d, 1 H) 7.02 - 7.19 (10H) 7.23 -7.27 (2 H); ¹³C-NMR (101 MHz, CDCl₃) δ [ppm] : 20.1 (t) 22.6 (q) 25.7 (q) 29.2 (t) 30.2 (t) 43.1 (t) 47.7 (d) 50.8 (d) 62.7 (d) 73.2 (s) 118.6 (d) 120.5 (s) 126.2 (d) 126.3 (d) 127.2 (d) 127.3 (d) 127.5 (s) 128.2 (d) 128.3 (d) 129.2 (d) 130.5 (d) 131.1 (d) 131.2 (d) 136.5 (s) 137.5 (s) 137.6 (s) 151.8 (s) 170.4 (s).
E) Zu einer Lösung von 16 g des vorstehend erhaltenen 2-(2,2-Dimethyl-1a,7b-dihydro-2*H*-oxireno[*c*]chromen-6-yl)-*N*-1,2,3,4-tetrahydronaphthalen-1-ylacetamids in 88 ml Ethanol wurden 88 ml einer 25%-igen, wäßrigen Ammoniaklösung zugegeben und es wurde 18 h lang bei RT gerührt. Anschließend gab man 200 ml Dichlormethan und 50 ml Methanol hinzu und ließ weitere 15 Min. lang rühren. Dann gab man 200 ml Wasser zu und ließ erneut 15 Min. lang rühren. Man trennte die organische Phase ab und dampfte sie unter vermindertem Druck weitgehend ein. Der verbliebene Rückstand wurde mit 30 ml EE gerührt, filtriert und am Rotationsverdampfer bei 70 °C und 20 bar getrocknet. Man erhielt 3,1 g 2-(4-Amino-3-hydroxy-2,2-dimethyl-3,4-dihydro-2H-chromen-6-yl)-N-1,2,3, 4-tetrahydronaphthalen-1-ylacetamid als grauen Feststoff, welcher ohne weitere Reinigung für die nachfolgende Umsetzung eingesetzt wurde. ¹H-NMR (400 MHz, DMSO-D₆) δ [ppm] : 1.08 (s, 6H) 1.35 (s, 6 H) 1.60 -1.95 (12H) 2.63 -2.83 (4H) 3.17 (d, 2H) 3.32 - 3.40 (4H) 3.49 (d, 2 H)4.90 - 4.98 (2 H) 5.32 - 5.38 (2 H) 6.62 (d, 2 H) 7.01 (dd, 2 H) 7.05 - 7.18 (8 H) 7.47 (d, 2 H) 8.32 -8.35 (2 H); ¹³C-NMR (101 MHz, DMSO-D₆) δ [ppm] : 18.6 (q) 19.9 (t) 27.0 (q) 28.7 (t) 29.8 (t) 41.7 (t) 46.2 (d) 50.8 (d) 76.6 (d) 77.8 (s) 115.6 (d) 125.5 (s) 125.6 (d) 125.7 (d) 126.5 (d) 127.9 (s) 128.0 (d) 128.1 (d) 128.3 (d) 128.4 (d) 128.6 (d) 136.9 (s) 137.5 (s) 150.5 (s) 169.8 (s).
F) 1,67 g 4-Ethylbenzolsulfonylchlorid wurden tropfenweise zu einer Lösung von 3,75 g des vorstehend erhaltenen 2-(4-Amino-3-hydroxy-2,2-dimethyl-3,4-dihydro-2H-chromen-6-yl)-*N*-1,2,3,4-tetrahydronaphthalen-1-ylacetamids und 8,4 ml Triethylamin in 160 ml Dichlormethan hinzugegeben. Man gab noch 5 ml Dimethylformamid (= DMF) hinzu und ließ das entstandene Gemisch 18 h lang bei RT rühren. Anschließend gab man 100 ml Wasser hinzu und ließ erneut 5 Min. lang rühren. Man trennte die organische Phase ab und dampfte das Lösungsmittel unter vermindertem Druck weitgehend ein. Der verbliebene Rückstand wurde an Kieselgel chromatographiert (mobile Phase: EE/Cyclohexan/Methanol 110:140:2 v/v/v) und die Produktphasen wurden vereinigt und eingeegengt. Der Rückstand wurde mit Petrolether/Diethylether 10:1 v/v gerührt Das entstandene Kristallisat wurde abgesaugt und am Rotationsverdampfer bei 40°C und 25 bar getrocknet. Es wurden 2,3 g der Titelverbindung als farbloses Kristallisat erhalten, ¹H-NMR (400 MHz, CDCl₃) δ [ppm] :m 1.14 (s, 3 H) 1.16 (s, 3 H) 1.26 (t, 3 H) 1.28 (t, 3 H) 1.44 (s, 6 H) 1.60 - 1.85 (8 H) 1.95 - 2.08 (2 H) 2.66 - 2.83 (8 H) 3.18 - 3.33 (6 H) 3.53 -3-63 (2 H) 4.18 - 4.28 (2 H) 5.02 -5.13 (2 H) 5.26 -5.37 (2 H) 5.52 -5.60 (2 H) 6.54 (d, 2 H) 6.66 - 6.71 (2 H) 6.95 - 7.19 (m, 10 H) 7.34 - 7.39 (4 H) 7.85 -7.92(4 H);¹³C-NMR (101 MHz, CDCl₃) δ [ppm] : 15.1 (q) 18.4 (q) 20.1 (t) 26.6 (q) 28.8 (t) 29.2 (t) 30.2 (t) 42.9 (t) 47.7 (d) 47.8 (d) 55.1 (d) 74.9 (d) 75.0 (d) 78.6 (s) 78.7 (s) 118.0 (d) 121.0 (s) 126.2 (d) 126.3 (d) 127.2 (s) 127.4 (d) 128.2 (d) 128.8 (d) 128.9 (d) 129.2 (d) 129.3 (d) 130.3 (d) 136.5 (s) 137.6 (s) 150.3 (s) 152.3 (s) 170.3 (s).

### Beispiel 2:

### 2-((3S,4R)-4-{[(4-Ethylphenyl)sulfonyl]amino}-3-hydroxy-2,2-dimethyl-3,4-dihydro-2H-chromen-6-yl)-N-[(1R)-1,2,3,4-tetrahydronaphthaten-1-yl]acetamid

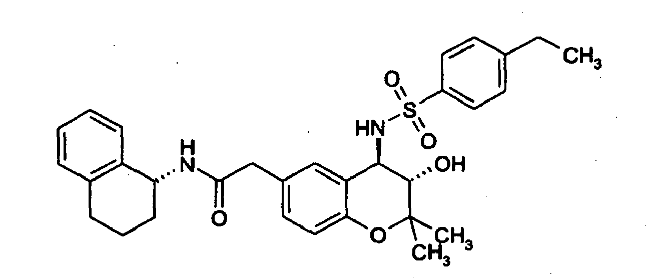

A) 24,5 g CDl, 30 g 2,2-(Dimethyl-2*H*-chromen-6-yl)essigsäure (Herstellung s. Beispiel 1B)) und 22,8 ml (1*R*)-1,2,3,4-Tetrahydro-1-naphthylamin wurden entsprechend der vorstehend in Beispiel 1C) angegebenen Weise umgesetzt. Man erhielt 49 g 2-(2,2-Dimethyl-2*H*-chromen-6-yl)-*N*-[(1*R*)-1,2,3,4-tetrahydonaphthalen-1-ylacetamid als farbloses Kristallisat, welches ohne weitere Reinigung für die nachfolgende Umsetzung eingesetzt wurde. ¹H-NMR (400 MHz, CDCl₃) δ [ppm]: 1.41 (s, 6 H) 1.60 - 1.85(3H) 1.98-2.08(1 H) 2.65 - 2.80 (2H) 3.45 - 3.55 (2 H) 5.12 - 5.20 (1H) 5.60 (d, 1H) 5.66 (d, 1H) 6.26 (d, 1H) 6.71 (d, 1H) 6.86 (d, 1H) 6.96 (dd, 1H) 7.02 - 7.07 (1H) 7.08 - 7.17 (3 H); ¹³C-NMR (101 MHz, CDCl₃) δ [ppm]: 20.2 (t) 28.0 (q, 2 C) 29.2 (t) 30.3 (t) 43.2 (t) 47.7 (d) 76.3 (s) 116.8 (d) 121.7 (s) 122.0 (d) 126.2 (d) 126.9 (s) 127.2 (d) 128.2 (d) 129.1 (d) 129.8 (d) 131.3 (d) 136.7 (s) 137.5 (s) 152.2 (s) 170.7 (s).
B) Zu einer Lösung von 44 g des vorstehend erhaltenen 2-(2,2-Dimethyl-2*H*-chromen-6-yl)-*N*-[(1*R*)-1,2,3,4-tetrahydronaphthaten-1-ylacetamids in 800 ml Dichlormethan gab man 5 g (S,S)-Mangan-(III)-salen und 7 g Pyridin-N-oxid hinzu. Man kühlte die so erhaltene Vorlage auf 0 °C und gab während 45 Min. eine Mischung aus 660 ml einer wäßrigen Natriumhypochloritlösung (CI > 13 %) und 88 ml einer 9 %-igen wäßrigen Lösung von Na₂HPO₄ hinzu. Es wurde weitere 3 h lang bei 0 °C gerührt, dann trennte man die organische Phase und rührte diese 1 h lang mit 500 g Celite^{®} 503. Man filtrierte vom Feststoff ab und wusch mit Dichlormethan nach bis das Filtrat farblos war. Das Filtrat wurde unter vermindertem Druck bis zur Trockene eingedampft. Man erhielt 40 g 2-[(1aS,7b*S*)-(2,2-Dimethyl-1a,7b-dihydro-2*H*-oxireno-[*c*]chromen-6-yl]-*N*-1,2,3,4-tetrahydronaphthalen-1-ylacetamid als rohes Öl, welches ohne weitere Reinigung oder Charakterisierung für die nachfolgende Umsetzung eingesetzt wurde.
C) 40 g des vorstehend erhaltenen 2-[(1a*S*,7b*S*)-(2,2-Dimethy)-1a,7b-dihydro-2*H-*oxireno[c]chromen-6-yl]-*N*-1,2,3,4-tetrahydronaphthalen-1-ylacetamids und 250 ml einer 25%-igen wäßrigen Ammoniaklösung wurden entsprechend der vorstehend in Beispiel 1E) angegebenen Weise umgesetzt. Chromatographie des Rohprodukts an Kieselgel (mobile Phase: Dichlormethan / Methanol / 25%-ige wäßrige Ammoniaklösung (75:50:2 v/v/v)) lieferte 13,2 g 2-[(3S,4R)-4-Amino-3-hydroxy-2,2-dimethyl-3,4-dihydro-2H-chromen-6-yl]-N-[(1R)-1,2,3,4-tetrahydronaphthalen-1-yl]acetamid als Öl, welches ohne weitere Reinigung für die nachfolgende Umsetzung eingesetzt wurde, ¹H-NMR (400 MHz, DMSO-D₆) δ [ppm] : 1.08 (s, 6 H) 1.35 (s, 6 H) 1.60 - 1.95 (12 H) 2.63 -2.83 (4 H) 3.17 (d, 2 H) 3.32 - 3.40 (4 H) 3.49 (d, 2 H) 4.90 - 4.98 (2H) 5.32-5.38 (2H) 6.62 (d, 2H) 7.01 (dd, 2 H) 7.05 - 7.18 (8 H) 7.47 (d, 2 H) 8.32 -8.35 (2H); ¹³C-NMR (101 MHz, DMSO-D₆) δ [ppm]: 18.6 (q) 19.9 (t) 27.0 (q) 28.7 (t) 29.8 (t) 41.7 (t) 46.2 (d) 50.8 (d) 76.6 (d) 77.8 (s) 115.6 (d) 125.5 (s) 125.6 (d) 125.7 (d) 126.5 (d) 127.9 (s) 128.0 (d) 128.1 (d) 128.3 (d) 128.4 (d) 128.6 (d) 136.9 (s) 137.5 (s) 150.5 (s) 169.8 (s).
D) 5,94 ml 4-Ethylbenzolsuffonylchlorid, 13,2 g des vorstehend erhaltenen 2-[(3S,4R)-4-Amino-3-hydroxy-2,2-dimethyl-3,4-dihydro-2H-chromen-6-yl]-N-[(1R)-1,2,3,4-tetrahydronaphthalen-1-yl]acetamid, 88 ml Triethylamin und 4 ml Dimethylformamid wurden entsprechend der vorstehend in Beispiel 1F) angegebenen Weise umgesetzt. Man erhielt 6,2 g der Titelverbindung als festen, amorphen Schaum; ¹H-NMR (400 MHz, CDCl₃) δ [ppm] : 1.15 (s, 3 H) 1.26 (t, *J*=7.6 Hz, 3 H) 1.45 (s, 3 H) 1.60 - 1.85 (3 H) 2.01 (m, 1 H) 2.65 - 2.82 (4 H) 3.20 - 3.33 (3 H) 3.58 (dd, *J*=9.0, 3.0 Hz, 1 H) 4.23 (dd, *J* =9.0, 8.7 Hz, 1 H) 5.04 (m, 1 H) 5.16 (d, *J*=8.7 Hz, 1 H) 5.51 (d, *J*=8.5 Hz, 1 H) 6.54 (d, *J*=2.0 Hz, 1 H) 6.68 (d, *J*=8.4 Hz, 1 H) 6.98 (dd, *J*=8.4, 2.0 Hz, 1 H) 6.95 - 7.19 (4 H) 7.37 (m, 2 H) 7.87 (m, 2 H); ¹³C-NMR (101 MHz, CDCl₃) δ [ppm] : 15.1 (q) 18.4 (q) 20.1 (t) 26.6 (q) 28.9 (t) 29.2 (t) 30.2 (t) 43.0 (t) 47.8 (d) 55.1 (d) 75.0 (d) 78.7 (s) 118.0 (d) 121.1 (s) 126.2 (d) 127.3 (s) 127.4 (d, 3 C) 128.2 (d) 128.7 (d) 129.0 (d, 2 C) 129.3 (d) 130.4 (d) 136.5 (s) 137.6 (s) 137.7 (s) 150.4 (s) 152.3 (s) 170.3 (s); =-8,3 ° (c=0.1, MeOH).

### Beispiel 3:

### N-Benzyl-2-{4-[[(4-ethylphenyl)sulfonyl](neopentyl)amino]-3-hydroxy-2,2-dimethyl-3,4-dihydro-2H-chromen-6-yl}acetamid

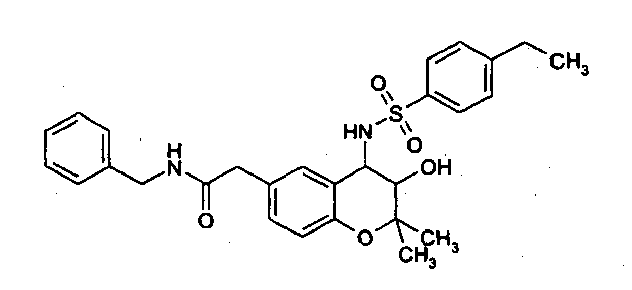

A) 16,2 g in 300 ml THF gelöstes CDI wurde langsam zu einer Lösung von 19,7 g 2,2-Dimethyl-2*H*-chromen-6-yl)essigsäure (Herstellung s. Beispiel 1 B)) in 300 ml THF hinzugegeben und 10 Min. lang bei RT gerührt. Zu dieser Vorlage tropfte man langsam 10,9 ml Benzylamin hinzu und rührte das entstandene Gemisch 1 h lang. Das Lösungsmittel wurde unter vermindertem Druck weitgehend eingedampft und der verbliebene Rückstand wurde einmal mit 500 ml einer Mischung aus EE und Wasser (2:3 v/v) extrahiert. Man dampfte die organische Phase unter vermindertem Druck weitgehend ein und chromatographierte den verbliebenen Rückstand an Kieselgel (mobile Phase: EE/Cyclohexan 1:1 v/v). Trocknung der Produktfraktionen am Rotationsverdampfer bei 70 °C und 20 bar lieferte 28 g *N*-Benzyl-2-(2,2-dimethyl-2H-chromen-6-yl)acetamid als Öl, welches ohne weitere Reinigung oder Charakterisierung für die nachfolgende Umsetzung eingesetzt wurde.
B) 980 ml einer gesättigten wäßrigen Natriumhydrogencarbonat-Lösung wurden zu einer Lösung von 28 g des vorstehend erhaltenen *N*-Benzyl-2-(2,2-dimethyl-2H-chromen-6-yl)acetamids in 480 ml Dichlormethan zugegeben. Zu dieser Vorlage gab man insgesamt 44,1 g MCPBA in drei Portionen von je 14,7 g im Abstand von jeweils 5 Min. hinzu und rührte 18 h lang bei RT. Die organische Phase wurde abgetrennt, einmal mit je 200 ml einer gesättigten wäßrigen Natriumhydrogencarbonat-Lösung extrahiert und die organische Phase unter vermindertem Druck weitestgehend eingedampft. Man erhielt 35 g *N*-Benzyl-2-(2,2-dimethyl-1a,7b-dihydro-2H-oxireno[*c*]chromen-6-yl)acetamid als rohes Öl, welches ohne weitere Reinigung oder Charakterisierung für die nachfolgende Umsetzung eingesetzt wurde.
C) Zu einer Lösung von 35 g des vorstehend erhaltenen *N*-Benzyl-2-(2,2-dimethyl-1a,7b-dihydro-2H-oxireno[c]chromen-6-yl)acetamids in 250 ml Ethanol wurden 250 ml einer 25%-igen wäßrigen Ammoniaklösung zugegeben und es wurde 18 h lang bei RT gerührt Das Reaktionsgemisch wurde in 500 ml Wasser gegossen und mit 250 ml Dichlormethan extrahiert. Die organische Phase wurde abgetrennt, über Natriumsulfat getrocknet und unter vermindertem Druck weitgehend eingedampft. Der verbliebene Rückstand wurde mit 200 ml Diethylether versetzt. Das nach einiger Zeit entstandene Kristallisat wurde abgesaugt und am Rotationsverdampfer bei 60 °C und 20 bar getrocknet. Man erhielt 11 g 2-(4-Amino-3-hydroxy-2,2-dimethyl-3,4-dihydro-2*H*-chromen-6-yl)-*N*-benzylacotamid als grauen Feststoff, welcher ohne weitere Reinigung oder Charakterisierung für die nachfolgende Umsetzung eingesetzt wurde.
D) Zu einer Lösung von 11 g des vorstehend erhaltenen 2-(4-Amino-3-hydroxy-2,2-dimethyl-3,4-dihydro-2*H*-chromen-6-yl)-*N*-benzylacetamids in 200 ml Methanol wurden 4 ml Trimethylacetaldehyd hinzugegeben. Man gab 2,44 g NaBH₃CN in mehreren Portionen zu und rührte die entstandene Suspension 2 h lang bei 50 °C. Nach Abkühlung auf RT wurde das Reaktionsgemisch in 200 ml Wasser gegossen. Man extrahierte die wäßrige Phase einmal mit 150 ml EE, trocknete die vereinigten organischen Phasen über Natriumsulfat und dampfte das Lösungsmittel unter verminder tem Druck weitgehend ein. Chromatographie des verbliebenen Rückstandes an Kieselgel (mobile Phase: EE/Cyclohexan 1:1 v/v) und Trocknung der Produktfraktionen am Rotationsverdampfer lieferte 10,8 g *N*-Benzyl-2-[3-hydroxy-2,2-dimethyl-4-(neopentylamino)-3,4-dthydro-2H-chromen-6-yl]acetamid als farbloses Öl, ¹H-NMR (400 MHz, CDCl₃) δ [ppm] : 1.15 (s, 3 H) 1.25 (t, 3 H) 1.43 (s, 3 H) 2.70 (q, 2 H) 3.18 - 3.31 (3 H) 3.55 (dd, 1 H) 4.22 (dd, 1 H) 4.30 (d, 2 H) 5.51 (d, 1 H) 5.81 (t, 1 H) 6.56 (d, 1 H) 6.67 (d, 1 H) 6.96 (dd, 1 H) 7.15 (m, 2 H) 7.21 - 7.30 (3 H) 7.32 (m, 2 H) 7.84 (m, 2 H); ¹³C-NMR (101 MHz, CDCl₃) δ [ppm] : 15.1 (q) 18.5 (q) 26.6 (q) 28.8 (t) 42.7 (t) 43.5 (t) 55.1 (d) 74.8 (d) 78.7 (s) 117.9 (d) 121.2 (s) 127.0 (s) 127.4 (d, 2 C) 127.5 (d, 3 C) 128.7 (d; 2 C) 128.9 (d, 3 C) 130.4 (d) 137.6 (s) 138.1 (s) 150.2 (s) 152.3 (s) 171.0 (s).

### Beispiel 4:

### N-Benzyl-2-{4-[[(4-ethylphenyl)sulfonyl](neopentyl)amino]-3-hydroxy-2,2-dimethyl-3,4-dihydro-2H-chromen-6-yl}acetamid

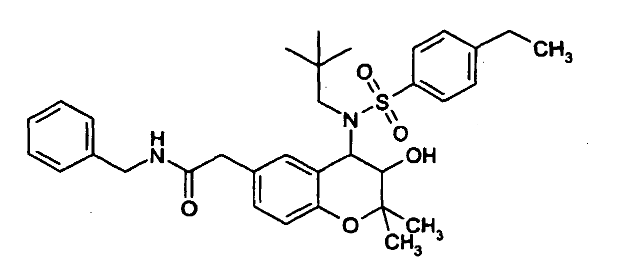

4,1 ml 4-Ethylbenzolsulfonylchlorid wurden tropfenweise zu einer Lösung von 10,8 g des vorstehend in Beispiel 3 erhaltenen *N*-Benzyl-2-[3-hydroxy-2,2-dimethyl-4-(neopentyl-amino)3,4-dihydro-2H-chromen-6-yl]acetamids und 5 ml Triethylamin in 100 ml Dichlormethan hinzugegeben. Man dampfte Dichlormethan umgehend unter vermindertem Druck weitestgehend ein und rührte das entstandene Reaktionsgemisch 90 Min. lang bei 65 °C und 180 bar. Man goß den gesamten Ansatz in 150 ml Wasser, extrahierte die wäßrige Phase einmal mit 200 ml EE. Man dampfte das Lösungsmittel unter vermindertem Druck weitgehend ein, trocknete den verbliebenen Rückstand über Natriumsulfat und chromatographierte den verbliebenen Rückstand an Kieselgel (mobile Phase: Petrolether/EE 3:1 v/v). Trocknung der Produktfraktionen im Ölpumpenvakuum lieferte 3,6 g der Titelverbindung (2 Konformere) als farbloses Öl, ¹H-NMR (400 MHz, CDCl₃) δ [ppm] : 0.75 (s, 9H) 1.15 (s, 3 H) 1.26 (t, 3 H) 1.46 (s, 3 H) 2.36 (1H) 2.65 - 2.75 (2 H) 2.90 -3.40 (4H) 3.86 (d,d 1 H) 4.39 (d, 2H) 4.76 (d, 1 H) 5.50 (t, 1 H) 6.48 (1 H) 6.73 (d, 1 H) 7.03 (dd, 1 H) 7.15 -7.35 (7 H) 7.81 (m, 2 H) **major**; ¹H-NMR (400 MHz, CDCl₃) δ [ppm]: 0.86 (s, 9H) 1.19 (s, 3 H) 1.24 (t, 3 H) 1.51 (s, 3 H) 2.65 - 2.75 (2 H) 2.81 (d, 1 H) 3.25 -3.60 (4H) 4.25 - 4.61 (4 H) 4.60 (d,d 1 H) 5.87 (t, 1 H) 6.73 (d, 1 H) 6.96 (dd, 1 H) 7.15 - 7.35 (8 H) 7.66 (m, 2 H) **minor**; ¹³C-NMR (101 MHz, CDCl₃) δ [ppm] : 15.1 (q) 17.9 (q) 27.0 (q) 28.8 (q, 3C) 28.8 (t) 32.0 (s) 43.0 (t) 43.7 (t) 56.3 (t) 59.0 (d) 71.2 (d) 79.1 (s) 118.4 (d) 120.7 (s) 127.0 -130.4 (12C) 137.2 (s) 138.1 (s) 150.4 (s) 153.0 (s) 170.7 (s) **major**; ¹³C-NMR (101 MHz, CDCl₃) δ [ppm] : 15.1 (q) 18.6 (q) 27.2 (q) 28.0 (q, 3C) 28.8 (t) 33.5 (s) 43.3 (t) 43.6 (t) 63.0 (t) 63.4 (d) 73.2 (d) 78.8 (s) 118.0 (d) 122.0 (s) 125.7 - 129.8 (12C) 137.3 (s) 138.3 (s) 150.2 (s) 151.8 (s) 171.3 (s) **minor**.

### Beispiel 5:

### N-(4-Chlorobenzyl)-2-(3-hydroxy-2,2-dimethyl-4-{[(3-methylphenyl)sulfonyl]amino}-3,4-dihydro-2H-chromen-6-yl)acetamid

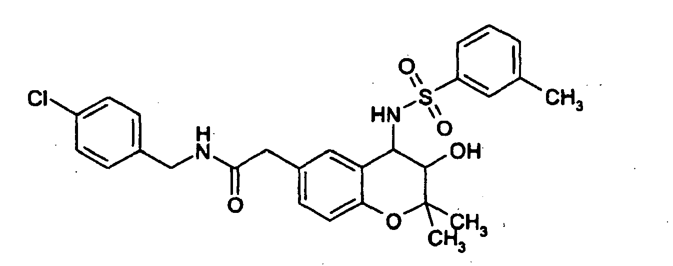

A) 175,6 g Methyl-4-hydroxyphenylacetat und 128,9 g Phenylboronsäure wurden in 3,5 Im-Xylol zusammengegeben. Zu diesem Gemisch gab man 88,9 g 3-Methylbut-2-enal und 130 ml Eisessig. Unter Stickstoffatmosphäre wurde in einer Dean-Stark-Apparatur bis zur etwa 70 %-igen Umsetzung des Phenols (etwa etwa 48 - 72 h) auf 140°C erhitzt. Anschließend ließ man das Reaktionsgemisch auf RT abkühlen, filtrierte und dampfte das Lösungsmittel unter vermindertem Druck ein. Der verbliebene Rückstand wurde in einer 1:1 Mischung aus THF und einer 25%-igen wäßrigen Ammoniaklösung (v/v) gelöst und 2 h lang gerührt. Man dampfte das THF unter vermindertem Druck weitgehend ab und gab EE zu. Die organische Phase wurde abgetrennt, der Reihe nach mit einer wäßrigen 1 N NaOH und einer gesättigten wäßrigen Kochsalzlösung gewaschen und schließlich über Natriumsulfat getrocknet. Man dampfte das Lösungsmittel unter vermindertem Druck weitgehend ein und chromatographierte den verbliebenen Rückstand an Kieselgel (mobile Phase: n-Hexan/EE 15:1 bis 10:1 v/v). Trocknung der Produktfraktionen im Ölpumpenvakuum lieferte 106 g Methyl-(2,2-dimethyl-2*H*-chromen-6-yl)acetat als blassgelbes Öl, welches ohne weitere Charakterisierung für die nachfolgende Umsetzung eingesetzt wurde.
B) 106 g des vorstehend erhaltenen Methyl-(2,2-dimethyl-2*H*-chromen-6-yl)acetats wurden in 900 ml THF gelöst. Zu dieser Vorlage gab man eine Lösung von 57,6 g LiOH in 900 ml Wasser und rührte 16 h lang bei RT. Das THF wurde unter vermindertem Druck weitgehend abgedampft und der wäßrige Rückstand wurde durch Zugabe einer wäßrigen 6N Salzsäurelösung angesäuert. Man extrahierte die wäßrige Phase mit EE und wusch die vereinigten organischen Phasen der Reihe nach mit einer gesättigten wäßrigen Kochsalzlösung und mit Wasser. Die organische Phase wurde über Natriumsulfat getrocknet und das Lösungsmittel im Vakuum weitestgehend eingedampft. Man erhielt 97,6 g 2,2-(Dimethyl-2*H*-chromen-6-yl)essigsäure, welche ohne weitere Reinigung oder Charakterisierung für die nachfolgende Umsetzung eingesetzt wurde.
C) 14,0 g der vorstehend erhaltenen 2,2-(Dimethyl-2*H*-chromen-6-yl)essigsäure und 13,54 g EDC×HCl wurden bei RT in Dichlormethan gelöst. Zu dieser Vorlage gab man unter Rühren 10,0 g 4-Chlorbenzylamin tropfenweise hinzu und rührte 16 h lang bei RT weiter. Anschließend wusch man das Reaktionsgemisch der Reihe nach mit Wasser, 1 N wäßriger Salzsäurelösung und gesättigter wäßriger Kochsalzlösung und trocknete die organische Phase über Natriumsulfat. Eindampfen des Lösungsmittels unter vermindertem Druck und Trocknen des verbliebenen Rückstandes im Ölpumpenvakuum lieferte 21,92 g des rohen N-4-Chlorbenzyl-2-(2,2-dimethyl-2H-chromen-6-yl)acetamids, welches ohne weitere Reinigung oder Charakterisierung für die nachfolgende Umsetzung eingesetzt wurde.
D) 700 ml einer gesättigten wäßrigen Natriumhydrogencarbonat-Lösung wurden zu einer Lösung von 21,92 g des vorstehend erhaltenen *N*-4-Chlorbenzyl-2-(2,2-dimethyl-2H-chromen-6-yl)acetamids in 600 ml Dichlormethan zugegeben. Zu dieser Vorlage gab man insgesamt 31,6 g MCPBA (72 %) portionsweise hinzu und rührte 16 h lang bei RT. Die organische Phase wurde abgetrennt, zweimal mit einer 5%-igen wäßrigen Natriumhydrogencarbonat-Lösung gewaschen, über Natriumsulfat getrocknet und unter vermindertem Druck weitestgehend eingedampft. Trocknung im Ölpumpenvakuum lieferte N-(4-chlorobenzyl)-2-(2,2-dimethyl-1a,7b-di-hydro-2H-oxireno[c]chromen-6-yl)acetamide als rohes Öl, welches ohne weitere Reinigung oder Charakterisierung für die nachfolgende Umsetzung eingesetzt wurde.
E) Das vorstehend erhaltene N-(4-Chlorobenzyl)-2-(2,2-dimethyl-1a,7b-dihydro-2H-oxireno[c]chromen-6-yl)acetamid wurde sofort in ein Gemisch von soviel Ethanol und 25%-ige wäßriger Ammoniaklösung (6:5 v/v) gegeben, daß eine 0,2 M Lösung der Verbindung erhalten wurde und es wurde 16 h lang bei 50 °C gerührt. Anschließend ließ man auf RT abkühlen und dampfte das Lösungsmittel unter vermindertem Druck weitgehend ein. Man chromatographierte den verbliebenen Rückstand an Kieselgel (mobile Phase: Gradient Dichlormethan /Methanal/25%-ige wäßrige Ammoniaklösung 97,5:2:0,5 bis 90:9,5:0,5 v/v/v). Trocknen der Produktfraktionen lieferte 7,3 g 2-(4-Amino-3-hydroxy-2,2-dimethyl-3,4-dihydro-2*H*-chromen-6-yl)-*N*-4-chlorbenzylacetamid, welches ohne weitere Reinigung für die nachfolgende Umsetzung eingesetzt wurde. Das andere Regioisomer, 2-[3-Amino-4-hydroxy-2,2-di-methylchromen-6-yl]-*N*-4-chlorbenzylacetamid, wurde nicht beobachtet.
F) Auf einem Probenplatz einer Probenplatte für die automatische Parallelsynthese wurden zu einer Lösung von 9 mg des vorstehend erhaltenen 2-(4-Amino-3-hydroxy-2,2-dimethyl-3,4-dihydro-2*H*-chromen-6-yl)-*N*-4-chlorbenzylacetamids in 0,5 ml Dichlormethan der Reihe nach 15 mg PS-Methylpiperidin und eine Lösung von 6,0 mg 3-Methylbenzolsulfonylchlorid in 0,5 ml Dichlormethan zugegeben. Man schüttelte 40 h lang bei RT und gab anschließend 20 mg AMPS zu. Es wurde weitere 16 h lang bei RT geschüttelt, bevor man die flüssige Reaktionsphase vorn Harz abtrennte und das Harz zweimal mit je 1 ml Dichlormethan wusch. Das Lösungsmittel der vereinigten organischen Phasen wurde unter vermindertem Druck abgedampft und man erhielt die Titelverbindung in einer Reinheit von 95 % (Bestimmung durch HPLC-MS), [M+H]⁺ 529.

### Beispiel 6:

### N-Butyl-2-{4-[[(2,5-dimethoxyphenyl)sulfonyl](2-ethylbutyl)amino]-3-hydroxy-2,2-dimethyl-3,4-dihydro-2H-chromen-6-yl}acetamid

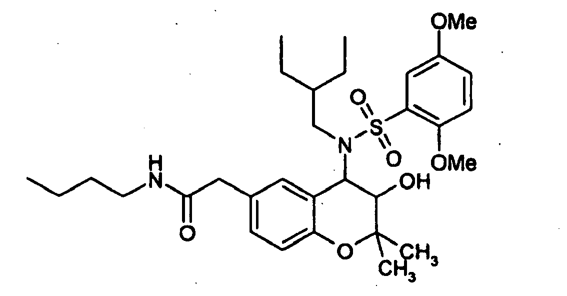

A) 10,0 g 2,2-(Dimethyl-2*H*-chromen-6-yl)essigsäure (Herstellung siehe Beispiel 5 B)), 9,67 g EDCxHCl und 5,41 g n-Butylamin wurden entsprechend der in Beispiel 5 C) angegebenen Weise umgesetzt. Man erhielt 17,5 g rohes N-(n-Butyl)-2-(2,2-dimethyl-2H-chromen-6-yl)acetamid, welches ohne weitere Reinigung oder Charakterisierung für die nachfolgende Umsetzung eingesetzt wurde.
B) 700 ml einer gesättigten wäßrigen Natriumhydrogencarbonat-Lösung wurden mit einer Lösung von 17,5 g des vorstehend erhaltenen *N*-(n-Butyl)-2-(2,2-dimethyl-2H-chromen-6-yl)acetamids in 600 ml Dichlormethan und 31,6 g MCPBA (72 %) entsprechend der in Beispiel 5 D) angegebenen Weise umgesetzt. Man erhielt das rohe N-Butyl-2-(2,2-dimethyl-1a,7b-dihydro-2H-oxireno[*c*]chromen-6-yl)acetamid, welches ohne weitere Reinigung oder Charakterisierung sofort für die nachfolgende Umsetzung eingesetzt wurde.
C) Das vorstehend erhaltene N-Butyl-2-(2,2-dimethyl-1a,7b-dihydro-2H-oxireno[c]-chromen-6-yl)acetamid wurde sofort in ein Gemisch von soviel Ethanol und 25%-ige wäßriger Ammoniaklösung (6:5 v/v) gegeben, daß eine 0,2 M Lösung der Verbindung erhalten wurde und entsprechend der in Beispiel 5 E) angegebenen Weise weiterbehandelt. Man erhielt 7,4 g 2-(4-Amino-3-hydroxy-2,2-dimethyl-3,4-dihydro-2H-chromen-6-yl)-*N*-(n-butyl)acetamid, welches ohne weitere Reinigung für die nachfolgende Umsetzung eingesetzt wurde. Das andere Regioisomer, 2-[3-Amino-4-hydroxy-2,2-dimethylchromen-6-yl)]-*N*-(n-butyl)acetamid, wurde nicht beobachtet.
D) 610 mg des vorstehend erhaltenen 2-(4-Amino-3-hydroxy-2,2-dimethyl-3,4-dihydro-2H-chromen-6-yl)-*N*-(n-butyl)acetamids wurden in 20 ml THF gelöst und es wurden 220 µl TMOF hinzugefügt. Anschließend gab man zu dieser Vorlage 197 mg Ethylbutyraldehyd und schüttelte das Reaktionsgemisch 16 h lang bei RT. Man entfernte das Lösungsmittel unter vermindertem Druck, nahm den verbliebenen Rückstand mit 20 ml Methanol auf, gab 7,9 g PS-BH₄ hinzu und schüttelte das Reaktionsgemisch weitere 16 h lang bei RT. Das Reaktionsgemisch wurde dann filtriert und das Reaktionsharz wurde mit Methanol gewaschen. Die vereinigten Filtrate wurden unter vermindertem Druck weitgehend eingedampft, der verbliebene Rückstand wurde in 20 ml Dichlormethan gelöst und es wurden der Reihe nach 0,4 Äquivalente eines an sich bekannten polymergebundenen Aldehyds (PS-CHO) und 0,6 Äquivalente AMPS zugegeben. Man schüttelte erneut 16 h lang bei RT, filtrierte die flüssige Phase vom Reaktionsharz ab und wusch dieses mit THF nach. Die vereinigten flüssigen organischen Phasen wurden unter vermindertem Druck eingedampft und man erhielt 572 mg zu 95 % reines (Bestimmung durch HPLC-MS) 2-[4-(2-Ethylbutylamino)-3-hydroxy-2,2-dimethylchromen-6-yl]-*N*-(n-butyl)acetamid welches ohne weitere Reinigung oder Charakterisierung für die nachfolgende Umsetzung eingesetzt wurde.
E) Auf einem Probenplatz einer Probenplatte für die automatische Parallelsynthese wurden zu einer Lösung von 14,8 mg des vorstehend erhaltenen 2-[4-(2-Ethyl-butylamino)-3-hydroxy-2,2-dimethylchromen-6-yl]-*N*-(n-butyl)acetamids in 0,6 ml Dichlormethan der Reihe nach 20 mg PS-Methylpiperidin-Harz und eine Lösung von 37,1 mg 2,5-Dimethoxybenzolsulfonylchlorid in 0,4 ml Dichlormethan zugegeben. Man schüttelte 168 h lang bei RT, filtrierte vom Harz ab und gab anschließend 120 mg PS-AMPS zum Filtrat hinzu. Es wurde weitere 16 h lang bei RT geschüttelt, bevor man die flüssige Reaktionsphase vom Harz abtrennte und das Harz zweimal mit je 1 ml Dichlormethan wusch. Das Lösungsmittel der vereinigten organischen Phasen wurde unter vermindertem Druck abgedampft und man erhielt die Titelverbindung in einer Reinheit von 96 % (Bestimmung durch HPLC-MS), [M+H]⁺ 591.

Nach den in den vorstehend angegebenen Beispielen beschriebenen oder nach hierzu analogen Verfahren können auch die in der nachfolgenden Tabelle 6 angegebenen Verbindungen der Formel I hergestellt werden:

**Tabelle 6: Weitere Verbindungen der Formel I:**

| **Bsp. Nr.** | **R¹** | **R²** | **R³** | **R⁴** | **R⁵** | **R⁶** | ***C-3** | ***C-4** |
|---|---|---|---|---|---|---|---|---|
| 7 | Me | Me | 4-Ethylphenyl | Neopentyl | H | Benzyl | S | R |
| 8 | Me | Me | 4-Ethylphenyl | Neopentyl | H | Benzyl | R | S |
| 9 | Me | Me | 4-Ethylphenyl | H | H | (S)-Tetrahydronaphthalen-1-yl | S | R |
| 10 | Me | Me | 4-Ethylphenyl | Neopentyl | H | Phenylethyl | trans | |
| 11 | Me | Me | 4-Ethylphenyl | H | H | Benzyl | S | R |
| 12 | Me | Me | 4-Ethylphenyl | H | H | Phenylethyl | trans | |
| 13 | Me | Me | 3-Fluorphenyl | H | H | (S)-Tetrahydronaphthalen-1-yl | trans | |
| 14 | Me | Me | Phenyl | H | H | (S)-Tetrahydronaphthalen-1-yl | trans | |
| 15 | Me | Me | 4-Methylphenyl | H | H | (S)-Tetrahydronaphthalen-1-yl | trans | |
| 16 | Me | Me | 4-Ethylphenyl | H | 4-Phenylpiperazin-1-yl | | trans | |
| 17 | Me | Me | 4-lodphenyl | H | | n-Butyl | trans | |
| 18 | Me | Me | 4-Methylphenyl | Cyclopropylmethyl | H | Benzyl | trans | |
| 19 | Me | Me | 4-Methylphenyl | 3-Methylbutyl | H | Benzyl | trans | |
| 20 | Me | Me | 2,5-Dimethoxyphenyl | 2-Ethylbutyl | H | n-Butyl | trans | |
| 21 | Me | Me | 2,5-Dimethoxyphenyl | 2-Ethylbutyl | H | 2-Furylmethyl | trans | |
| 22 | Me | Me | 3-Methoxyphenyl | n-Pentyl | H | 1,2-Dimethylpropyl | trans | |
| 23 | Me | Me | 4-Trifluormethylphenyl | 3-Methylbutyl | H | Benzyl | trans | |
| 24 | Me | Me | 3-Chlor-4-fluorphenyl | 2-Methyl-propyl | H | n-Propyl | trans | |
| 25 | Me | Me | 2-Naphthyl | 3-Methylbutyl | H | Benzyl | trans | |
| 26 | Me | Me | 4-Biphenyl | 3-Methylbutyl | H | n-Propyl | trans | |
| 27 | Me | Me | 3-Methoxyphenyl | 3-Methylbutyl | H | 4-Chlorbenzyl | trans | |

| | | | | | | | | |
|---|---|---|---|---|---|---|---|---|
| trans = trans-Stellung der Substituenten an C-3 und C-4, jedoch Gemisch der Stereoisomeren; Me = Methyl; "S" und "R" beziehen sich jeweils auf die Absolutkonfiguration am entsprechenden Kohlenstoff. | | | | | | | | |

### Beispiel I:

### 2-(4-{[(4-Ethylphenyl)sulfonyl]amino}-3-hydroxy-2,2-dimethyl-3,4-dihydro-2H-chromen-6-yl)-N-1,2,3,4-tetrahydronaphthalen-1-ylacetamid enthaltende Kapseln:

Es werden Kapseln in folgender Zusammensetzung pro Kapsel hergestellt:

| | |
|---|---|
| 2-(4-{[(4-Ethylphenyl)sulfonyl]amino}-3-hydroxy-2,2-dimethyl-3,4-dihydro-2*H*-Chromen-6-yl)-*N*-1,2,3,4-tetrahydronaphthalen-1-ylacetamid | 20 mg |
| Maisstärke | 60 mg |
| Milchzucker | 300 mg |
| EE | q.s. |

Der Wirkstoff, die Maisstärke und der Milchzucker werden unter Zuhilfenahme von EE zu einer homogenen pastösen Mischung verarbeitet. Die Paste wird zerkleinert und das entstehende Granulat wird auf ein geeignetes Blech gebracht und zur Entfernung des Lösungsmittels bei 45°C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann in 400 mg fassende Kapseln (= Kapselgröße 0) abgefühlt.

## Patentansprüche

1. Verbindungen der allgemeinen Formel I, 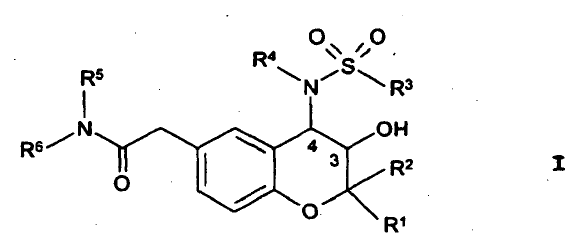 worin,
R¹ C₁₋₄-Alkyl bedeutet,
R² C₁₋₄-Alkyl bedeutet,
R³ Phenyl, welches gegebenenfalls 1-2-fach durch Halogen, C₁₋₄-Alkyl), C₁₋₄-Alkoxy oder Trifluormethyl substituiert ist; Naphthyl oder Biphenyl bedeutet,
R⁴ Wasserstoff; C₁₋₆-Alkyl oder C₃₋₇Cycloalkyl-C₁₋₄-alkyl bedeutet,
R⁵ Wasserstoff bedeutet und
R⁶ C₁₋₆-Alkyl; Phenyl-C₁₋₄-alkyl, dessen Phenylgruppe gegebenenfalls 1-fach durch Halogen substituiert ist; Furyl-C₁₋₄-alkyl oder Tetrahydronaphthyl bedeutet, oder
R⁵ und R⁶ gemeinsam mit dem Stickstoff, woran sie gebunden sind, einen Piperazinring bilden, welcher gegebenenfalls durch Phenyl substituiert ist.

2. Verbindungen nach Anspruch 1, worin R¹ und R² jeweils Methyl bedeuten.

3. Verbindungen nach Anspruch 1, worin R³ gegebenenfalls 1-fach substituiertes Phenyl bedeutet.

4. Verbindungen nach Anspruch 1, worin R⁴ Wasserstoff, C₁₋₆-Alkyl oder Cyclopropyl-C₁₋₄-alkyl bedeutet.

5. Verbindungen nach Anspruch 1, worin R⁶ Phenyl-C₁₋₄-alkyl oder Tetrahydronaphthyl bedeutet.

6. Verbindungen nach Anspruch 1, worin im Pyranring der den Hydroxy-Substituenten tragende Kohlenstoff (C-3) die S-Konfiguration aufweist und der den stickstoffhaltigen Substituenten tragende Kohlenstoff (C-4) die R-Konfiguration aufweist.

7. Verbindungen der Formel I nach einem der vorstehenden Ansprüche, welche ausgewählt sind aus der Gruppe bestehend aus
2-(4-{[(4-Ethylphenyl)sulfonyl]amino}-3-hydroxy-2,2-dimethyl-3,4-dihydro-2*H*-chromen-6-yl)-*N*-1,2,3,4-tetrahydronaphthalen-1-ylacetamid;
2-((3S,4R)-4-{[(4-Ethylphenyl)sulfonyl]amino}-3-hydroxy-2,2-dimethyl-3,4-dihydro-2H-chromen-6-yl)-N-[(1R)-1,2,3,4-tetrahydronaphthalen-1-yl]acetamid;
*N*-Benzyl-2-{4-[[(4-ethylphenyl)sulfonyl](neopentyl)amino]-3-hydroxy-2,2-dimethyl-3,4-dihydro-2*H*-chromen-6-yl}acetamid;
2-{4-[[(4-Ethylphenyl)sulfonyl](neopentyl)amino]-3-hydroxy-2,2-dimethyl-3,4-dihydro-2*H* chromen-6-yl)-*N*-(2-phenylethyl)acetamid und
2-(4-{[(4-Methylphenyl)sulfonyl]amino}-3-hydroxy-2,2-dimethyl-3,4-dihydro-2*H*-chromen-6-yl)-*N*-1,2,3,4-tetrahydronaphthalen-1-ylacetamid.

8. Arzneimittel, enthaltend eine pharmakologisch wirksame Menge einer Verbindung der Formel I nach Anspruch 1 und übliche pharmazeutische Hilfs- und/oder Trägerstoffe.

9. Verwendung von Verbindungen der Formel I nach Anspruch 1 für die Herstellung von Arzneimitteln zur Behandlung von Herz-Kreislauferkrankungen und/oder zur Behandlung von proliferativen, chronisch entzündlichen und Autoimmunerkrankungen.

10. Verfahren zur Herstellung von Verbindungen der Formel I, 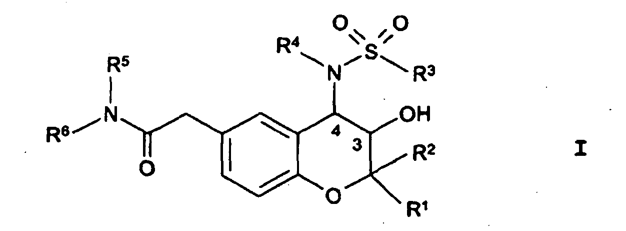 worin,
R¹ C₁₋₄-Alkyl bedeutet,
R² C₁₋₄-Alkyl bedeutet,
R³ Phenyl, welches gegebenenfalls 1-2-fach durch Halogen, C₁₋₄-Alkyl, C₁₋₄-Alkoxy oder Trifluormethyl substituiert ist; Naphthyl oder Biphenyl bedeutet,
R⁴ Wasserstoff; C₁₋₆-Alkyl oder C₃₋₇-Cycloalkyl-C₁₋₄-alkyl bedeutet,
R⁵ Wasserstoff bedeutet und
R⁶ C₁₋₆-Alkyl; Phenyl-C₁₋₄-alkyl, dessen Phenylgruppe gegebenenfalls 1-fach durch Halogen substituiert ist; Furyl-C₁₋₄-alkyl oder Tetrahydronaphthyl bedeutet, oder
R⁵ und R⁶ gemeinsam mit dem Stickstoff, woran sie gebunden sind, einen Piperazinring bilden, welcher gegebenenfalls durch Phenyl substituiert ist,
**dadurch gekennzeichnet, daß** man eine Verbindung der allgemeinen Formel II, 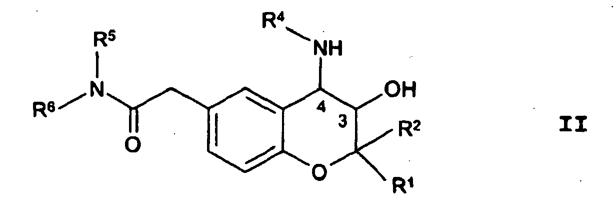 worin R¹, R², R⁴, R⁵, und R⁶ obige Bedeutungen besitzen, mit einer Verbindung der allgemeinen Formel III,
X-SO₂-R³ III
worin R³ obige Bedeutung besitzt und X eine abspaltbare Fluchtgruppe bedeutet, umsetzt.

11. Verbindungen der allgemeinen Formel II, 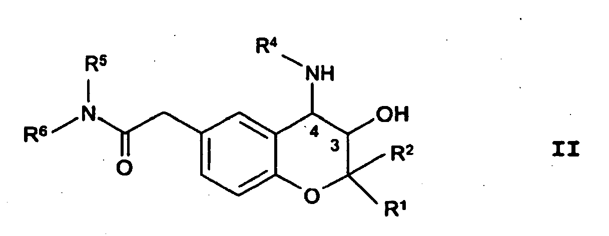 worin
R¹ C₁₋₄-Alkyl bedeutet,
R² C₁₋₄-Alkyl bedeutet,
R⁴ Wasserstoff; C₁₋₆-Alkyl oder C₃₋₇-Cycloalkyl-C₁₋₄-alkyl bedeutet,
R⁵ Wasserstoff bedeutet und
R⁶ C₁₋₆-Alkyl; Phenyl-C₁₋₄-alkyl, dessen Phenylgruppe gegebenenfalls 1-fach durch Halogen substituiert ist; Furyl-C₁₋₄-alkyl oder Tetrahydronaphthyl bedeutet, oder
R⁵ und R⁶ gemeinsam mit dem Stickstoff, woran sie gebunden sind, einen Piperazinring bilden, welcher gegebenenfalls durch Phenyl substituiert ist.

## Claims

1. Compounds of the general formula I, 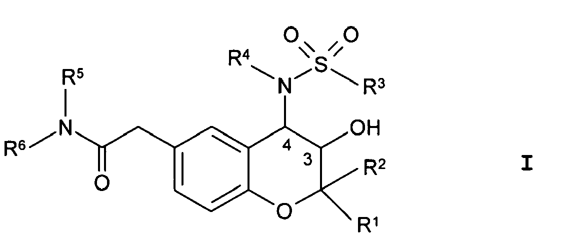 wherein
R¹ is C₁₋₄-alkyl,
R² is C₁₋₄-alkyl,
R³ is phenyl which is optionally substituted 1 to 2 times by halogen, C₁₋₄-alkyl, C₁₋₄-alkoxy or trifluoromethyl; naphthyl or biphenyl,
R⁴ is hydrogen; C₁₋₆-alkyl or C₃₋₇-cycloalkyl-C₁₋₄-alkyl,
R⁵ is hydrogen, and
R⁶ is C₁₋₆-alkyl; phenyl-C₁₋₄-alkyl, the phenyl group of which is optionally substituted once by halogen; furyl-C₁₋₄-alkyl or tetrahydronaphthyl, or
R⁵ and R⁶, together with the nitrogen to which they are bonded, form a piperazine ring which is optionally substituted by phenyl.

2. Compounds according to Claim 1, wherein R¹ and R² are each methyl.

3. Compounds according to Claim 1, wherein R³ is optionally once-substituted phenyl.

4. Compounds according to Claim 1, wherein R⁴ is hydrogen, C₁₋₆-alkyl or cyclopropyl-C₁₋₄-alkyl.

5. Compounds according to Claim 1, wherein R⁶ is phenyl-C₁₋₄-alkyl or tetrahydronaphthyl.

6. Compounds according to Claim 1, wherein in the pyran ring the carbon bearing the hydroxy substituent (C-3) is in the S configuration and the carbon bearing the nitrogen-containing substituent (C-4) is in the R configuration.

7. Compounds of Formula I according to one of the preceding claims, which are selected from the group consisting of
2-(4-{[(4-ethylphenyl)sulphonyl]amino}-3-hydroxy-2,2-dimethyl-3,4-dihydro-2*H-*chromen-6-yl)-N-1,2,3,4-tetrahydronaphthalen-1-ylacetamide;
2-((3S,4R)-4-{[(4-ethylphenyl)sulphonyl]amino}-3-hydroxy-2,2-dimethyl-3,4-dihydro-2H-chromen-6-yl)-N-[(1R)-1,2,3,4-tetrahydronaphthalen-1-yl]acetamide;
*N*-benzyl-2-{4-[[(4-ethylphenyl)sulphonyl](neopentyl)amino]-3-hydroxy-2,2-dimethyl-3,4-dihydro-2*H*-chromen-6-yl}acetamide;
2-{4-[[(4-ethylphenyl)sulphonyl](neopentyl)amino]-3-hydroxy-2,2-dimethyl-3,4-dihydro-2H-chromen-6-yl}-*N*-(2-phenylethyl)acetamide and
2-(4-{[(4-methylphenyl)sulphonyl]amino}-3-hydroxy-2,2-dimethyl-3,4-dihydro-2*H-*chromen-6-yl)-*N*-1,2,3,4-tetrahydronaphthalen-1-ylacetamide.

8. Medicament, containing a pharmacologically effective amount of a compound of Formula I according to Claim 1 and conventional pharmaceutical auxiliaries and/or carriers.

9. Use of compounds of Formula I according to Claim 1 for the manufacture of medicaments for the treatment of cardiovascular diseases and/or for the treatment of proliferative, chronic inflammatory and autoimmune diseases.

10. A process for the preparation of compounds of Formula I, 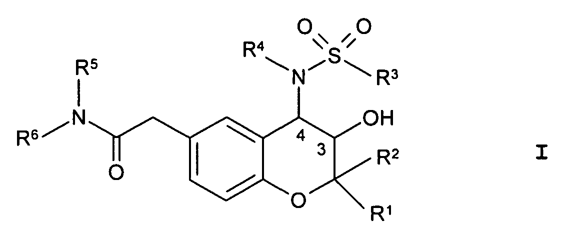 wherein
R¹ is C₁₋₄-alkyl,
R² is C₁₋₄-alkyl,
R³ is phenyl which is optionally substituted 1 to 2 times by halogen, C₁₋₄-alkyl, C₁₋₄-alkoxy or trifluoromethyl; naphthyl or biphenyl,
R⁴ is hydrogen; C₁₋₆-alkyl or C₃₋₇-cycloalkyl-C₁₋₄-alkyl,
R⁵ is hydrogen, and
R⁶ is C₁₋₆-alkyl; phenyl-C₁₋₄-alkyl, the phenyl group of which is optionally substituted once by halogen; furyl-C₁₋₄-alkyl or tetrahydronaphthyl, or
R⁵ and R⁶, together with the nitrogen to which they are bonded, form a piperazine ring which is optionally substituted by phenyl,
**characterised in that** a compound of the general formula II, 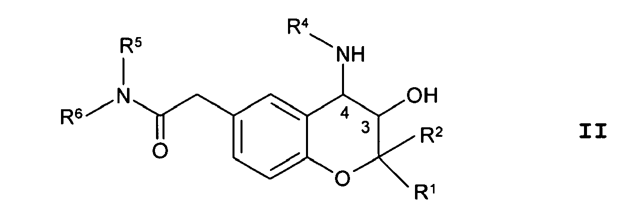 wherein R¹, R², R⁴, R⁵ and R⁶ have the above meanings, is reacted with a compound of the general formula III,
X-SO₂-R³ III
wherein R³ has the above meaning and X is a cleavable leaving group.

11. Compounds of the general formula II, 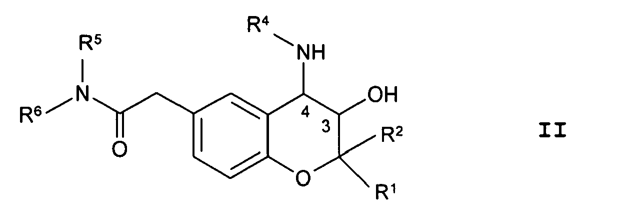 wherein
R¹ is C₁₋₄-alkyl,
R² is C₁₋₄-alkyl,
R⁴ is hydrogen; C₁₋₆-alkyl or C₃₋₇-cycloalkyl-C₁₋₄-alkyl,
R⁵ is hydrogen, and
R⁶ is C₁₋₆-alkyl; phenyl-C₁₋₄-alkyl, the phenyl group of which is optionally substituted once by halogen; furyl-C₁₋₄-alkyl or tetrahydronaphthyl, or
R⁵ and R⁶, together with the nitrogen to which they are bonded, form a piperazine ring which is optionally substituted by phenyl.

## Revendications

1. Composés de formule générale I, 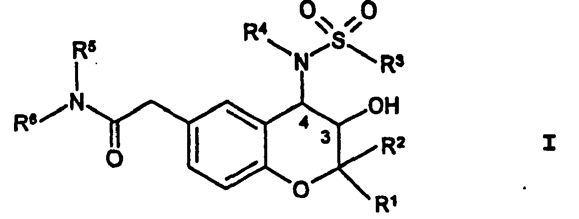 dans laquelle
R¹ est un groupe alkyle en C₁₋₄,
R² est un groupe alkyle en C₁₋₄,
R³ est un groupe phényle qui est éventuellement substitué une ou deux fois par des substituants halogéno, alkyle en C₁₋₄, alcoxy en C₁₋₄ ou trifluorométhyle ; ou un groupe naphtyle ou biphényle,
R⁴ est un atome d'hydrogéne, un groupe alkyle en C₁₋₆ ou (cycloalkyle en C₃-₇)(alkyle en C₁₋₄),
R⁵ est un atome d'hydrogène, et
R⁶ est un groupe alkyle en C₁₋₆; phényle (alkyle en C₁₋₄) dont le groupe phényle est éventuellement une fois substitué par un halogène ; furyle (alkyle en C₁₋₄) ou tétrahydronaphtyle, ou bien
R⁵ et R⁶, avec atome d'azote auquel ils sont liés, forment un noyau pipérazine, qui éventuellement porte un ou plusieurs substituants phényle.

2. Composés selon la revendication 1, R¹ et R² représentent chacun le groupe méthyle.

3. Composés selon la revendication 1, R³ est un groupe phényle éventuellement monosubstitué.

4. Composés selon la revendication 1, R⁴ est un atome d'hydrogène ou un groupe alkyle en C₁₋₆ ou cyclopropyle (alkyle en C₁₋₄).

5. Composés selon la revendication 1, R⁶ est un groupe phényle (alkyle en C₁₋₄) ou tétrahydronaphtyle.

6. Composés selon la revendication 1, dans lesquels, dans le noyau pyranne, l'atome de carbone C-3 portant le substituant hydroxy présente la configuration S, et l'atome de carbone C-4 portant le substituant azoté présente la configuration R.

7. Composés de formule I selon l'une des revendications précédentes, choisis dans l'ensemble consistant en les composés suivants :
2-(4-{[(4-éthylphényl)sulfonyl]amino} -3-hydroxy-2,2-diméthyl-3,4-dihydro-2*H*-chroméne-6-yl)-*N*-1,2,3,4-tétrahydronaphtaléne-1-ylacétalmide ;
2-((3S,4R)-4-{[(4-éthylphényl)sulfonyl]amino}-3-hydroxy-2,2-diméthyl-3,4-dihydro-2H-chromène-6-yl)-N-[(1R)-1,2,3,4-tétrahydronaphtalène-1-yl]acétamide ;
*N*-benzyl-2-{4-[[(4-éthylphényl)sulfonyl]-(néopentyl)amino]-3-hydroxy-2,2-diméthyl-3,4-dihydro-2*H*-chromène-6-yl}acétamide ;
2-{4-[[(4-éthylphényl)sulfonyl](néopentyl)amino]-3-hydroxy-2,2-diméthyl-3,4-dihydro-2*H*-chromène-6-yl}-*N*-(2-phényléthyl)acétamide ; et
2-(4-{[(4-méthylphényl)sulfonyl]amino}-3-hydroxy-2,2-diméthyl-3,4-dihydro-2*H*-chromène-6-yl)-*N*-1,2,3,4-tétrahydronaphtalène-1-ylacétamide.

8. Médicament, contenant une quantité à effet pharmacologique d'un composé de formule I selon la revendication 1 et des adjuvants et/ou excipients pharmaceutiques usuels.

9. Utilisation de composés de formule I selon la revendication 1 pour préparer des médicaments destinés au traitement de maladies cardiovasculaires et/ou pour le traitement de maladies prolifératives, inflammatoires chroniques et auto-immunes.

10. Procédé de préparation de composés de formule I 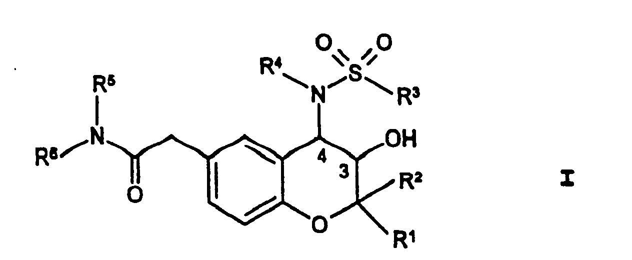 dans laquelle
R¹ est un groupe alkyle en C₁₋₄,
R² est un groupe alkyle en C₁₋₄,
R³ est un groupe phényle qui est éventuellement substitué une ou deux fois par des substituants halogéno, alkyle en C₁₋₄, alcoxy en C₁₋₄ ou trifluorométhyle ; ou un groupe naphtyle ou biphényle,
R⁴ est un atome d'hydrogène, un groupe alkyle en C₁₋₆ ou (cycloalkyle en C₃₋₇)(alkyle en C₁₋₄),
R⁵ est un atome d'hydrogène, et
R⁶ est un groupe alkyle en C₁₋₆; phényle (alkyle en C₁₋₄) dont le groupe phényle est éventuellement une fois substitué par un halogène ; furyle (alkyle en C₁₋₄) ou tétrahydronaphtyle, ou bien
R⁵ et R⁶, avec atome d'azote auquel ils sont liés, forment un noyau pipérazine, qui éventuellement porte un ou plusieurs substituants phényle.
**caractérisé en ce qu'**on fait réagir un composé de formule générale II, 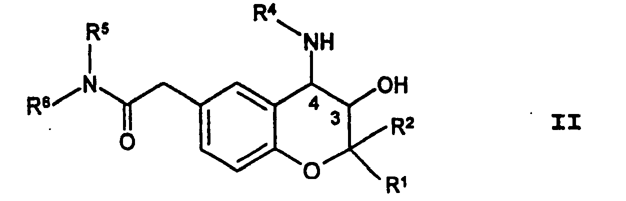 dans laquelle R¹, R², R⁴, R⁵ et R⁶ ont les significations ci-dessus, avec un composé de formule générale III,
X-SO₂-R³ III
dans laquelle R³ a les significations ci-dessus et X est un groupe labile.

11. Composés de formule générale II 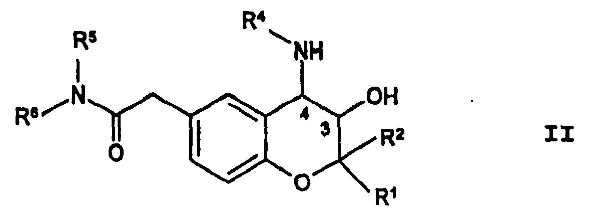 dans laquelle
R¹ est un groupe alkyle en C₁₋₄,
R² est un groupe alkyle en C₁₋₄,
R⁴ est un atome d'hydrogène ou un groupe alkyle en C₁₋₆ ou (cycloallcyle en C₃₋₇)(alkyle en C₁₋₄),
R⁵ est un atome d'hydrogène, et
R⁶ est un groupe alkyle en C₁₋₆ ; phényl (alkyle en C₁₋₄) dont le groupe phényle est éventuellement monohalogéné ; furyle (akyle en C₁₋₄) ou tétrahydronaphtyle, ou bien
R⁵ et R⁶, avec l'atome d'azote auquel ils sont liés, ferment un noyau pipérazine qui est éventuellement substitué par un ou des substituants phényle.
